(19) **Europäisches Patentamt**
**European Patent Office**
**Office européen des brevets**

(11) **EP 1 814 895 B1**

(12) **EUROPEAN PATENT SPECIFICATION**

(45) Date of publication and mention
of the grant of the patent:
**10.08.2011 Bulletin 2011/32**

(51) Int Cl.:
*C07H 19/00* (2006.01)    *A61K 31/70* (2006.01)
*C12Q 1/68* (2006.01)    *C12N 15/11* (2006.01)

(21) Application number: **05852038.8**

(22) Date of filing: **21.11.2005**

(86) International application number:
**PCT/US2005/042385**

(87) International publication number:
**WO 2006/060246 (08.06.2006 Gazette 2006/23)**

(54) **APPARATUS AND SYSTEM HAVING DRY CONTROL GENE SILENCING COMPOSITIONS**

VORRICHTUNG UND SYSTEM MIT TROCKENER KONTROLLGEN-VERSTUMMUNGSZUSAMMENSETZUNG

APPAREIL ET SYSTÈME CONTENANT DES COMPOSITIONS DE SILENÇAGE GÉNIQUE DE CONTROLE SÉCHÉS

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.11.2004 US 630320 P**
**04.05.2005 US 678165 P**
**18.11.2005 US 283483**
**18.11.2005 US 283481**
**18.11.2005 US 283482**
**18.11.2005 US 283484**

(43) Date of publication of application:
**08.08.2007 Bulletin 2007/32**

(73) Proprietor: **Dharmacon, Inc.**
**Lafayette, CO 80026 (US)**

(72) Inventors:
• **ROBERTSON, Barbara**
**Boulder, Colorado 80304 (US)**
• **LEAKE, Devin**
**Denver, Colorado 80238 (US)**
• **ROBINSON, Kathryn**
**Golden, Colorado 80403 (US)**
• **MARSHALL, William, S.**
**Boulder, CO 80303 (US)**
• **KHVOROVA, Anastasia**
**Boulder, CO 80305 (US)**

(74) Representative: **Maughan, Sophie Louise et al**
**Scott & York**
**Intellectual Property Ltd**
**45 Grosvenor Road**
**St Albans, Hertfordshire AL1 3AW (GB)**

(56) References cited:
**WO-A-2005/097992    WO-A2-2004/009847**
**US-A1- 2004 137 064**

• **SILVA J M ET AL: "RNA interference microarrays: High-throughput loss-of-function genetics in mammalian cells" PROCEEDINGS OF THE NATIONAL ACADEMY OF SCIENCES OF USA, NATIONAL ACADEMY OF SCIENCE, WASHINGTON, DC, US, vol. 101, no. 17, 27 April 2004 (2004-04-27), pages 6548-6552, XP002350199 ISSN: 0027-8424**
• **YOSHIKAWA T ET AL: "Transfection microarray of human mesenchymal stem cells and on-chip siRNA gene knockdown" JOURNAL OF CONTROLLED RELEASE, ELSEVIER, AMSTERDAM, NL, vol. 96, no. 2, 28 April 2004 (2004-04-28), pages 227-232, XP004502172 ISSN: 0168-3659**
• **"Complete Toolkit for Transfection Optimization." AMBION TECHNOTES, vol. 12, no. 3, 2005, page 6, XP002455826**
• **"Silencer Validated siRNAs Individual siRNAs Verified to Induce Gene Silencing" AMBION CATALOGUE, 2004, pages 10-11, XP002455827**

**(Cont. next page)**

- ERFLE HOLGER ET AL: "Reverse transfection on cell arrays for high content screening microscopy." NATURE PROTOCOLS 2007, vol. 2, no. 2, 2007, pages 392-399, XP002455828 ISSN: 1750-2799
- BAILEY S.N. ET AL.: 'Applications of transfected cell microarrays in high-throuhgput drug discovery' DRUG DISCOVERY TODAY vol. 7, 2002, pages S113 - S118, XP008026706
- KUMAR R. ET AL.: 'High-throughput selection of effective RNAi probes for gene silencing' GENOME RESEARCH vol. 13, 2003, pages 2333 - 2340, XP002292586
- MOUSSES S. ET AL.: 'RNAi microarray analysis in cultured mammalian cells' GENOME RESEARCH vol. 13, 2003, pages 2341 - 2347, XP009045304
- HONMA ET AL.: 'The role of atelocollagen-based cell transfection array in high-throughput screening of gene functions and in drug discovery' CURRENT DRUG DISCOVERY TECHNOLOGIES vol. 1, December 2004, pages 287 - 294, XP008092990
- ERFLE H ET AL: "SIRNA CELL ARRAYS FOR HIGH-CONTENT SCREENING MICROSCOPY", BIOTECHNIQUES, INFORMA LIFE SCIENCES PUBLISHING, WESTBOROUGH, MA, US, vol. 37, no. 3, 1 September 2004 (2004-09-01), pages 454-462, XP008068476, ISSN: 0736-6205
- "SuperArray Bioscience s siRNA Array Plates and SureSilencing siRNA and Antibody Kits, and Ambion s Silencer Phosphodiesterase siRNA Library", RNAI NEWS, 19 March 2004 (2004-03-19), Retrieved from the Internet: URL: http://www.genomeweb.com/rnai/superarr ay-bioscience-s-sirna-array-plates-and-sur esilencing-sirna-and-antibody->
- 'Product Guide 2004: Section 9.1', QIAGEN
- SureSilcencing Array Plates VALIDATED GENE-SPECIFIC siRNA FOR PATHWAY PROFILING BY REVERSE TRANSFECTION USER MANUAL FROM SUPERARRAY BIOSCIENCE CORPORATION VERSION 1.1 3/5/2004

**Description**

**CROSS-REFERENCE TO RELATED APPLICATIONS**

This United States Patent Application claims benefit of U.S. Provisional Application Serial No. 60/630,320, filed November 22, 2004, and U.S. Provisional Application Serial No. 60/678,165, filed May 04, 2005. This application also claims priority to United States Utility Patent Application Serial No. Unknown, Attorney Docket No. 16542.1.3 (Publication No: US-2006-0166234), entitled "APPARATUS AND SYSTEM HAVING DRY CONTROL GENE SILENCING COMPOSITIONS," filed on November 18, 2005; United States Utility Patent Application Serial No. Unknown, Attorney Docket No. 16542.1.1 (Publication No: US-2006-0115461), entitled "APPARATUS AND SYSTEM HAVING DRY GENE SILENCING COMPOSITIONS," filed on November 18, 2005; United States Utility Patent Application Serial No. Unknown, Attorney Docket No. 16542.1.2 (Publication No: US-2006-0110829), entitled "APPARATUS AND SYSTEM HAVING DRY GENE SILENCING POOLS," filed on November 18, 2005; and United States Utility Patent Application Serial No. Unknown, Attorney Docket No. 16542.1.4 (Publication No: US-2006-0110766), entitled "METHOD OF DETERMINING A CELLULAR RESPONSE TO A BIOLOGICAL AGENT," filed on November 18, 2005.

**BACKGROUND OF THE INVENTION**

**1. The Field of the Invention**

The present invention relates to an apparatus and system for use in RNA interference. More particularly, the present invention relates to an apparatus and system that include a well plate having control siRNA.

**2. The Related Technology**

Recently, a natural cellular regulatory pathway was discovered that uses transcribed microRNA ("miRNA") in order to control protein production. The miRNA includes a duplex region of sense and antisense RNA. This regulatory pathway uses miRNA in order to target complementary mRNA to inhibit production of the encoded protein. Accordingly, a complex series of proteins are involved in this RNA interfering pathway to inhibit or stop production of the proteins encoded by the mRNA. As such, the process is referred to as RNA interference or RNAi.

Additionally, it has been found that the RNAi pathway can be used with synthetic dsRNA (*e.g.*, siRNA) for silencing genes and inhibiting protein expression. This can allow for siRNA having specific sequences to be produced to target complementary DNA and/or mRNA encoding a specific protein. The siRNA can interact with the natural RNAi pathway to silence a target gene and inhibit production of the encoded polypeptide. The ability to silence a specific gene and inhibit production of the encoded protein has been used for basic research of gene function, gene mapping, cellular pathway analysis, and other gene-related studies.

In order to induce gene silencing, the siRNA needs to be introduced into a cell. While the most common procedures for introducing nucleic acids into cells has been forward transfection, reverse transfection ("RTF") has been developed more recently and used as an alternative to forward transfection procedures. In certain versions of RTF protocols, a complex of lipid-nucleic acid (*e.g.*, lipoplex) can be prepared and introduced into the test wells of a well plate. Cells are introduced into the test wells with the lipid-nucleic acid complexes, and incubated so that the siRNA can enter the cells. Examples of some RTF protocols can be found in U.S. Patent Numbers 5,811,274 to Palsson, 5,804,431 to Palsson and 6,544,790 to Sabatini and in U.S. Published Applications 2002/0006664 to Sabatini and 2003/070642 to Caldwell et al. As described in these references, RTF procedures for nucleic acids generally can have fewer steps compared to traditional forward transfection and may offer benefits in attempting to isolate the transfected cells to particular regions of a single surface, such as a glass slide. However, RTF procedures for siRNA have not been optimized to the point of practical application, and improvements in gene silencing efficacy are still needed, especially for situations in which one is experimenting with multiple different siRNAs, different gene targets or different cell lines. Silva J M ET AL: "RNA interference microarrays: High-throughput loss-of-function genetics in mammalian cells" PROCEEDINGS OF THE NATIONAL ACADEMY OF SCIENCES OF USA, NATIONAL ACADEMY OF SCIENCE, WASHINGTON, DC, US, vol. 101, no. 17, 27 April 2004 (2004-04-27), pages 6548-6552, discloses printing of siRNAs on to glass slides, followed by reverse transfection of cells by placing the spotted array in a tissue culture dish containing cells. A document entitled "SureSilencing Array Plates VALIDATED GENE-SPECIFIC siRNA FOR PATHWAY PROFILING BY REVERSE TRANSFECTION USER MANUAL" from Superarray Bioscience Corporation Version 1.1 3/5/2004 discloses array plates having wells pre-coated with a matrix containing siRNA duplex and transfection reagent. Cells are seeded with medium into pre-coated wells.

Often, RTF protocols can be performed on well plates in a manner that is not optimized or produces inaccurate and unreliable data. The lack of optimization can cause variations between plates, and can reduce the reliability of the data. Variations in data from a lack of optimization or an error can produce results that appear to be related to the gene silencing obtained from the siRNA, and are difficult to detect without comparing the test results to other experiments performed with the same siRNA. Additionally, systematic variations in data that occur can arise from the RTF conditions, and may be related to the amount of siRNA, amount of siRNA carrier, cell density, media, temperature, or various other factors that affect gene silencing.

Therefore, it would be advantageous to have an improved RTF protocol for testing the efficacy of gene silencing. Additionally, it would be beneficial to have an RTF format that uses controls to test the efficacy of gene silencing.

**BRIEF SUMMARY OF THE INVENTION**

Generally, embodiments of the present invention include well plates, kits, systems, and methods of using the same for testing the efficacy of gene silencing. Accordingly, the present invention provides well plates, kits, and systems that implement an improved RTF testing protocol for delivering control siRNA into cells to test the efficacy of gene silencing in other cells in the well plate or other well plates. The control siRNA can provide an indication of gene silencing efficacy that can be compared to known functionalities and standard results obtained from using the control siRNA in optimal or other test conditions. According to the invention, there is provided a reverse transfection plate in accordance with Claim 1, a kit in accordance with Claim 22, a method of testing the efficacy of gene silencing in accordance with Claim 23, and a method for making a reverse transfection plate in accordance with Claim 32.

In one embodiment, the present invention can include a reverse transfection plate for testing the efficacy of gene silencing. The plate can include at least a first control well including a substantially dry first control composition having at least a first control siRNA. The first control siRNA can be capable of providing a first indication of the gene silencing efficacy. Additionally, the first control composition can be configured such that the first control siRNA is capable of being solubilized or suspended in an aqueous medium in an amount sufficient for transfecting cells in the first control well. The control siRNA can be any one of a transfection control siRNA, positive control siRNA, or negative control siRNA. Optionally, the total amount of control siRNA in the first control composition can be present in an amount for transfecting cells in only the first control well.

In one embodiment, the plate can further include at least a second control well including a substantially dry second control composition. The second control composition can include at least a second control siRNA, which can be any of the transfection, positive, or negative control siRNAs. Preferably, the second control siRNA is different from the first control siRNA. As such, the second control siRNA can provide a second indication of gene silencing efficacy that is different from the first indication. The second control composition can be configured such that the second control siRNA is capable of being solubilized or suspended in an aqueous medium in an amount sufficient for transfecting cells in the second control well. Optionally, the first control composition includes a positive control siRNA and the second control composition includes a negative control siRNA. Alternatively, the first control composition includes a positive control siRNA and the second control composition includes a transfection control siRNA. In yet another alternative, the first control composition can have a transfection control siRNA and the second control composition can include a negative control siRNA.

In one embodiment, the plate can further include at least a third control well including a substantially dry third control composition. The third control composition can include at least a third control siRNA, which can be any of the transfection, positive, or negative control siRNA. Preferably, the third control siRNA is different from the first control siRNA and second control siRNA. As such, the third control siRNA can provide a third indication of gene silencing efficacy that is different from the first indication and second indication. The third control composition can be configured such that the third control siRNA is capable of being solubilized or suspended in an aqueous medium in an amount sufficient for transfecting cells in the third control well. Preferably, the first, second, and third control siRNAs include a transfection, positive, and negative control siRNA, respectively.

In one embodiment, the transfection control siRNA, positive control siRNA, or negative control siRNA can conform to the descriptions provided herein and in the references cited herein.

In one embodiment, the present invention provides a kit or system that includes a well plate having a control well with a substantially dry control composition comprised of control siRNA. Additionally, such a kit or system includes a polynucleotide carrier. The polynucleotide carrier can be a cationic lipid, polymer, lipopolymer, or the like. Additionally, the kit or system can include various solubilizing solutions, reagents, cell culture media, and the like.

In one embodiment, the present invention includes a method of testing the efficacy of gene silencing with control siRNA. This can include testing the conditions used in the RTF protocol, which may be related to cell density, type of polynucleotide carrier, carrier concentration, siRNA concentration, RTF protocol, or other factors that can alter the effectiveness for siRNA to silence genes. Additionally, such a testing method can include the use of any well plate consistent with the foregoing characterizations. Accordingly, an aqueous medium can be added to a first control well in the well plate, wherein the first control well includes a first control siRNA. The aqueous medium can solubilize or suspend the first control siRNA. Optionally, the aqueous medium can include a polynucleotide carrier such as a cationic lipid, polymer, lipopolymer, and the like, which can form a complex with the control siRNA. The complex can be prepared so as to be capable of being suspended or solubilized in the aqueous medium.

Cells can be added to the first well under conditions that permit transfection with the complex. The cells can be added in an amount of about $1x10^3$ to about $3.5x10^4$ or about $2x10^3$ to about $3x10^4$ cells per about 0.3 cm$^2$ to about 0.35 cm$^2$ of cell growth surface area. Subsequently, the control siRNA can contact the cell in a manner that allows for entry into the cellular cytoplasm. However, any mode of transfection can be used to cause the control siRNA to enter the cell. The well plate can then be maintained under conditions so that cell growth, cell division, transfection, and/or gene silencing occurs. After a proper duration that allows for transfection and/or the control siRNA to silence a known gene, the effect of the first control siRNA on the cells can be determined. The effect of the first control siRNA in the cells can be compared with a known effect of the first control siRNA.

In one embodiment, a second control siRNA can be used to test the efficacy of gene silencing. As such, the testing protocol can include adding the aqueous medium to a second control well in the well plate, wherein the second control well includes a second control siRNA. Subsequently, the cells can be added to the second control well under conditions that permit transfection, and the second control siRNA can then be transfected into the cells by any mode of transfection. The effect of the second control siRNA on the cells can be determined. The effect of the second control siRNA can be compared to the effect of the first control siRNA.

In one embodiment, a third control siRNA can be used to test the efficacy of gene silencing. As such, the testing protocol can include adding the aqueous medium to a third control well in the well plate, wherein the third control well includes a third control siRNA. Subsequently, the cells can be added to the third control well under conditions that permit transfection, and the third control siRNA can then be transfected into the cells by any mode of transfection. The effect of the third control siRNA on the cells can be determined. The effect of the third control siRNA can be compared to the effect of the first control siRNA and/or the effect of the second control siRNA.

In one embodiment, a blank well that is substantially devoid of siRNA can be used to test the efficacy of gene silencing. As such, the testing protocol can include adding an aqueous medium to a blank well in the well plate, the blank well being devoid of siRNA. Optionally, the aqueous medium can include the polynucleotide carrier. Optionally, the cells are then added to the blank well, and the effects of control siRNA on the cells are compared to the cells added to the blank well. On the other hand, the cells may not be added to the blank well so that it can be used for various calibrations.

These and other embodiments and features of the present invention will become more fully apparent from the following description and appended claims, or may be learned by the practice of the invention as set forth hereinafter.

## BRIEF DESCRIPTION OF THE DRAWINGS

To further clarify the above and other advantages and features of the present invention, a more particular description of the invention can be rendered by reference to specific embodiments thereof which are illustrated in the appended drawings. It is appreciated that these drawings depict only typical embodiments of the invention and are therefore not to be considered limiting of its scope. The invention can be described and explained with additional specificity and detail through the use of the accompanying drawings.

Figures 1A and 1B are schematic diagrams that illustrate an embodiment of a multi-well plate having control siRNA and test siRNA.

Figures 2A-2D are schematic diagrams that illustrate embodiments of arrangements of control siRNA on a multi-well plate

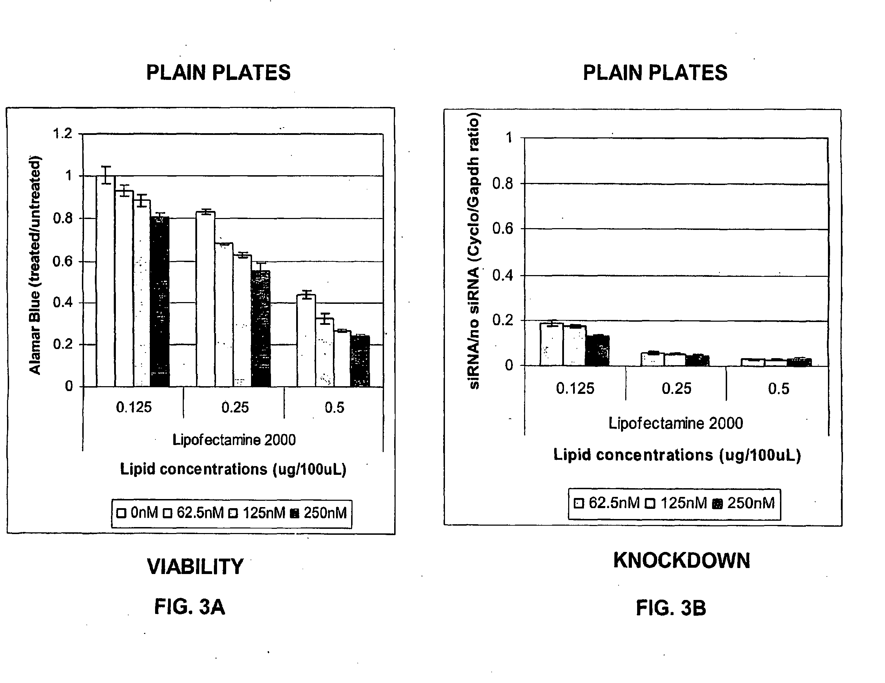

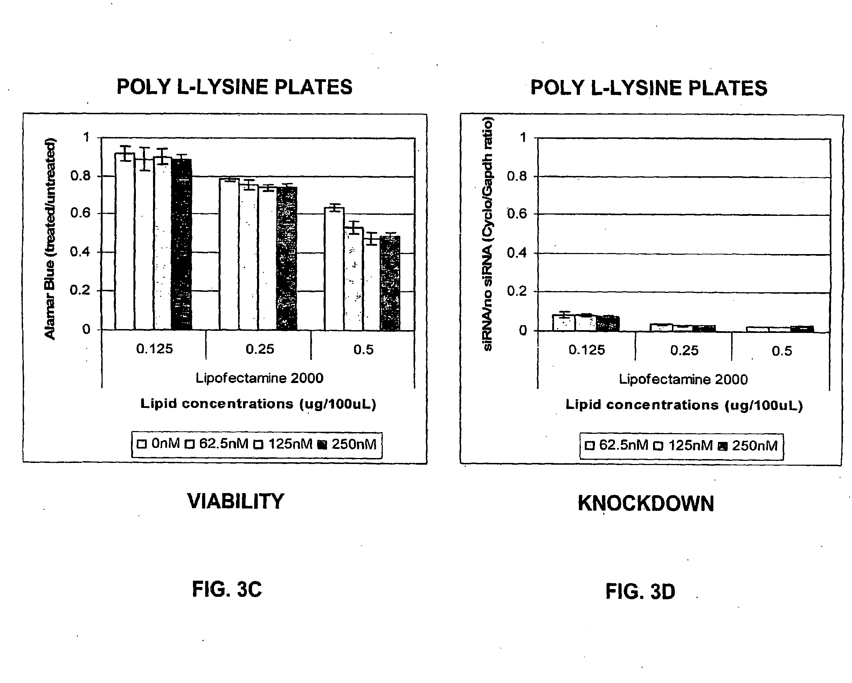

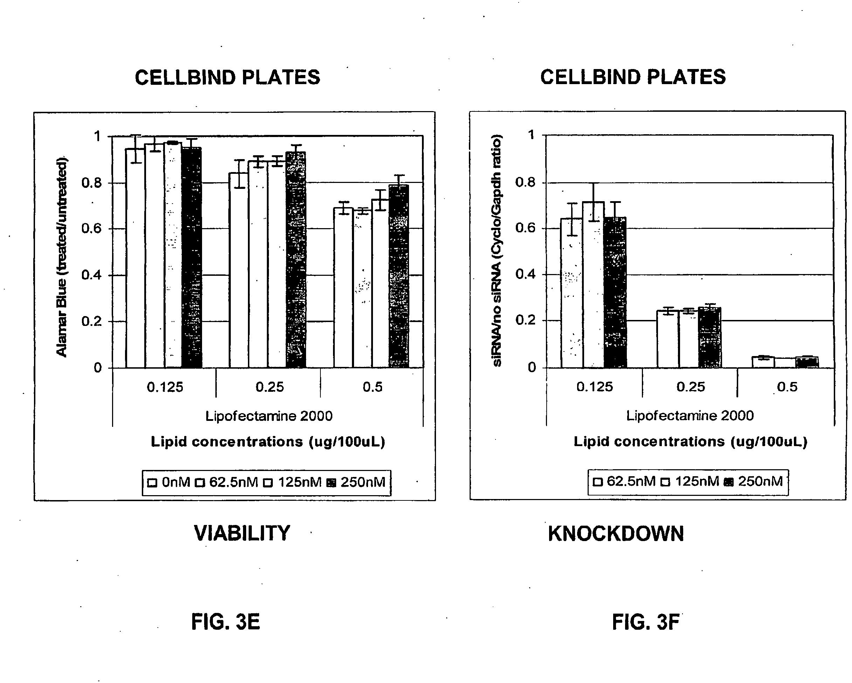

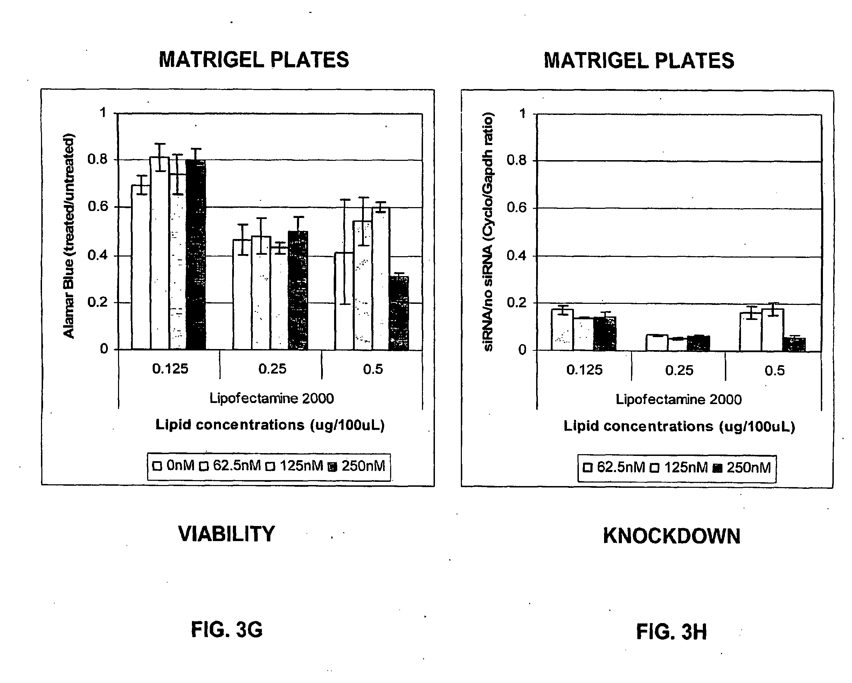

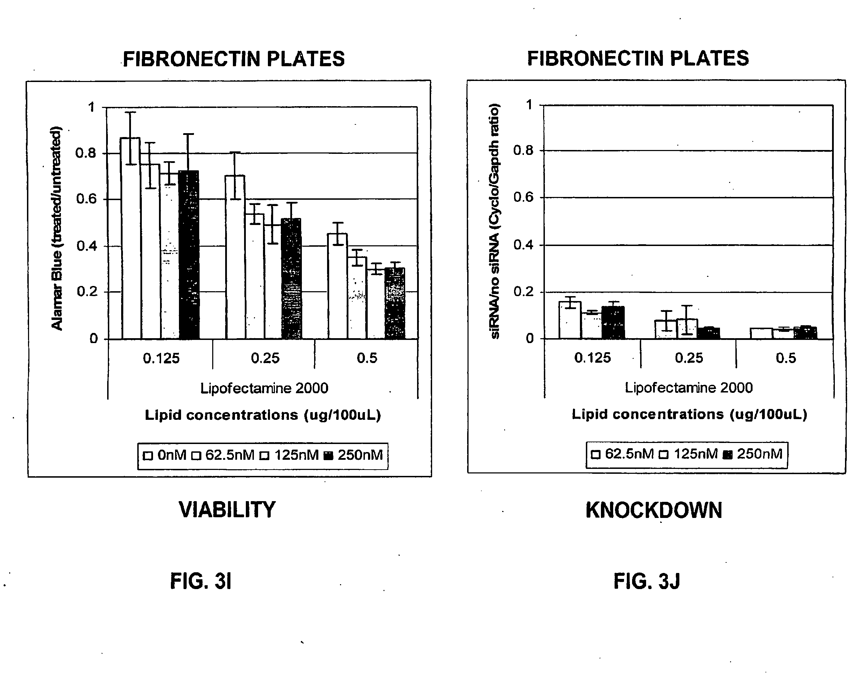

Figures 3A-3J are graphical representations of embodiments of the effects of various plate conditions on siRNA RTF gene silencing and cell viability. Figure 3A illustrates cell viability and Figure 3B illustrates gene silencing of plain plates. Figure 3C illustrates cell viability and Figure 3D illustrates gene silencing of poly-L-lysine ("PLL") coated plates. Figure 3E illustrates cell viability and Figure 3F illustrates gene silencing of CELLBIND™ plates. Figure 3G illustrates cell viability and Figure 6H illustrates gene silencing of MATRIGEL™ plates. Figure 3I illustrates cell viability and Figure 3J illustrates gene silencing of fibronectin plates. In cell survival measurements, the Y-axis represents relative levels of survival with 1.0 being 100% viability. In gene silencing measurements, the Y-axis represents the level of gene expression compared to controls with 1.0 being 100% expression, and "ug" is microgram.

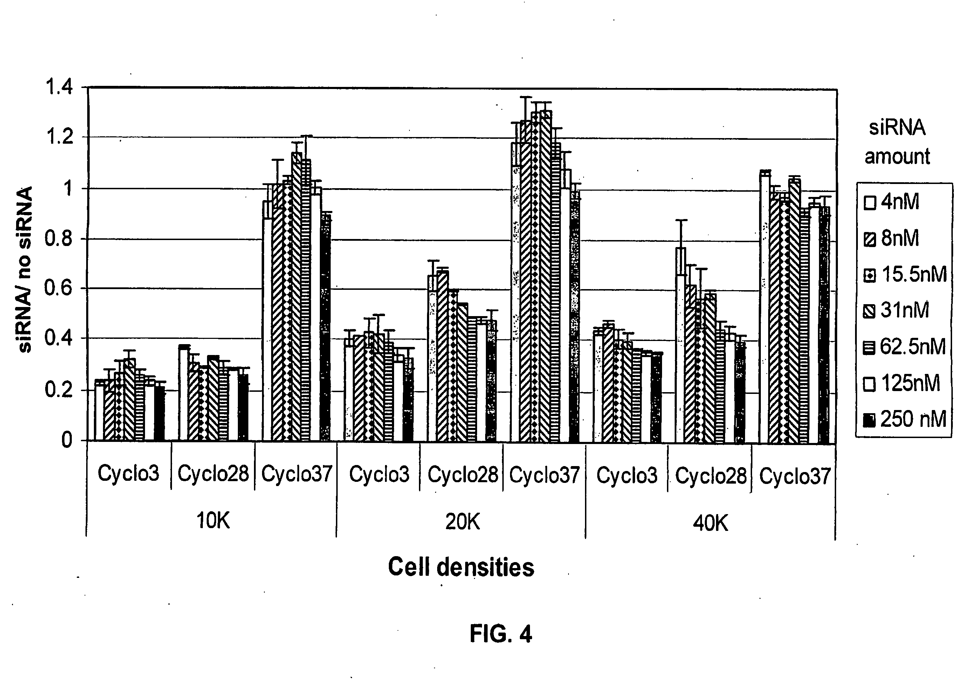

Figure 4 is a graphical representation of an embodiment of a comparison of human cyclophilin B gene silencing at different cell plating densities. HeLa cells at 10K, 20K, and 40K cells per well were transfected with cyclo 3, cyclo 28, or cyclo 37 siRNA at varying concentrations (*e.g.*, 4 nM-250 nM) using Dharma*FECT*™ 1 lipid at 0.063 micrograms ("ug") for 10K cells, 0.125 ug for 20K cells, and 0.250 ug for 40K cells of per 100 microliters ("uL") volume, respectively. The total volume in each well is 125 uL. The figure demonstrates the role that cell density plays in siRNA silencing efficiency in a reverse transfection format. For gene silencing, the Y-axis represents the level of gene expression compared to controls with 1.0 being 100% expression.

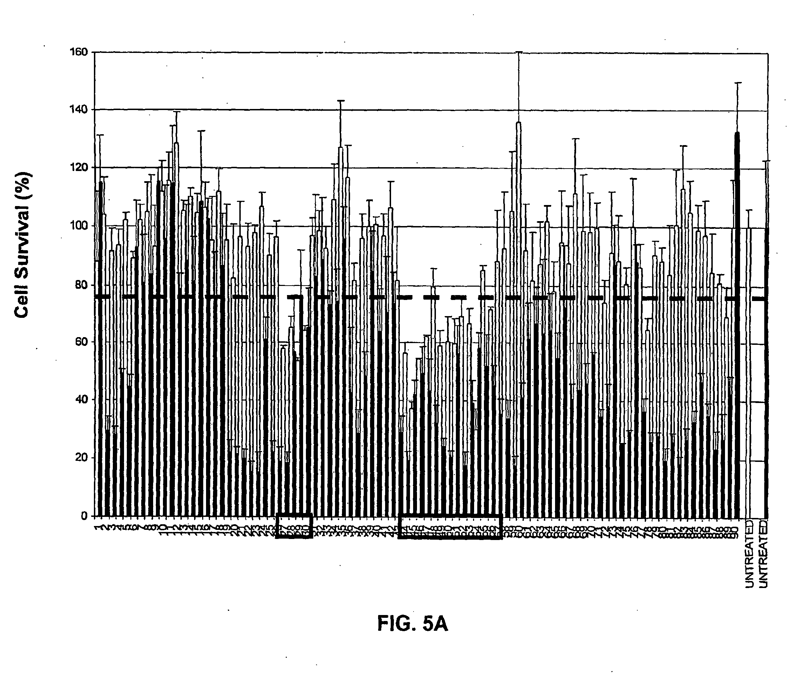

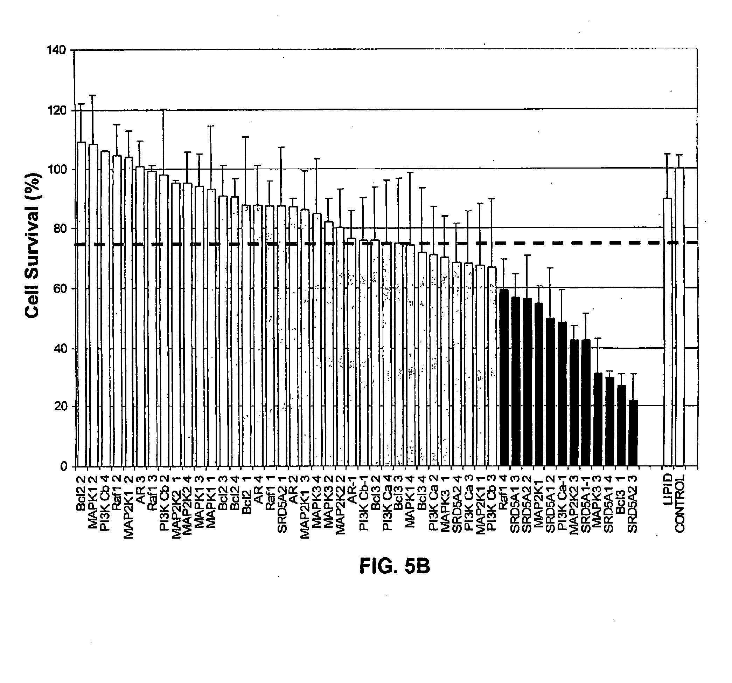

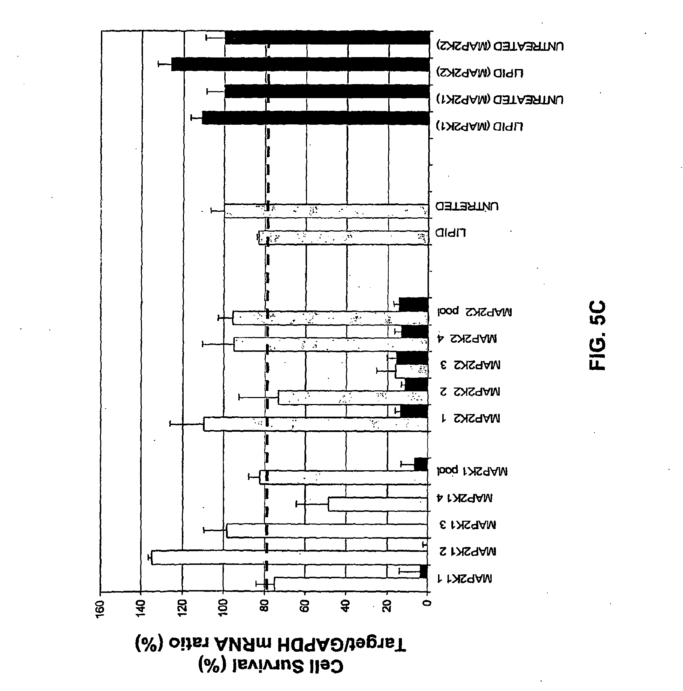

Figures 5A-5C are graphical representations of an embodiment of the identification of toxic siRNA. HeLa cells were forward transfected at 5,000 cells per well with 10 nM siRNA. Figure 5A depicts a DBI-siRNA walk identifying toxic siRNA, wherein the black bars represent DBI silencing, and the gray bars represent cell survival. Figure 5B depicts cell survival resulting from the introduction of one of forty-eight different siRNA directed against one of twelve different targets. Figure 5C depicts an examination of eight siRNA derived from Figure 5B, and shows that toxicity is unrelated to target specific silencing. Also, the data demonstrates that pooling is one means of eliminating siRNA-induced toxicity.

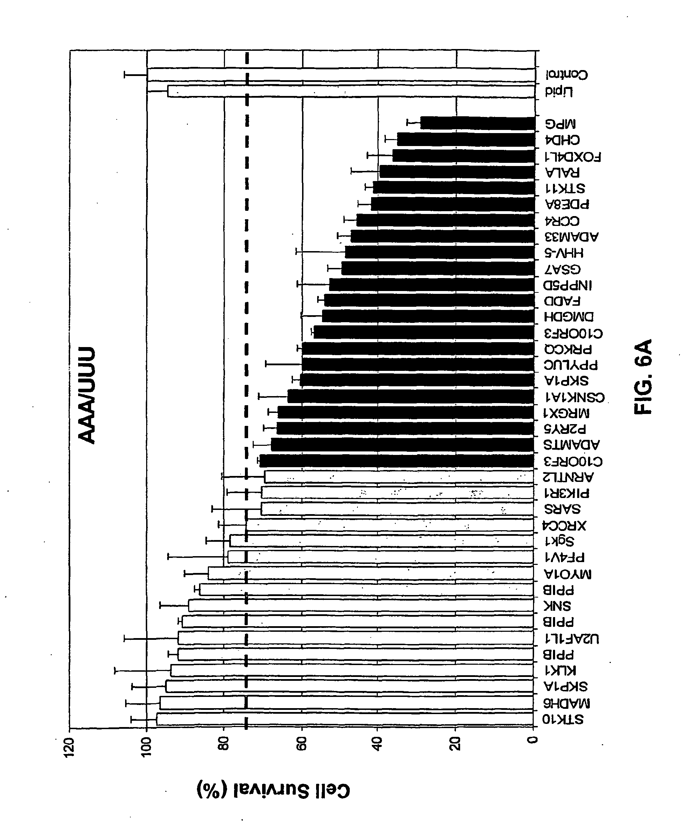

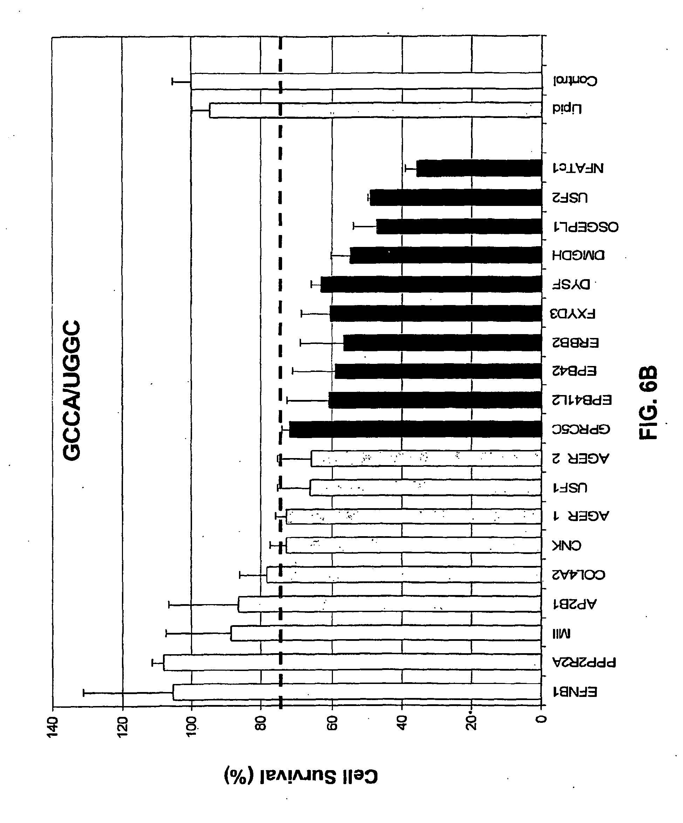

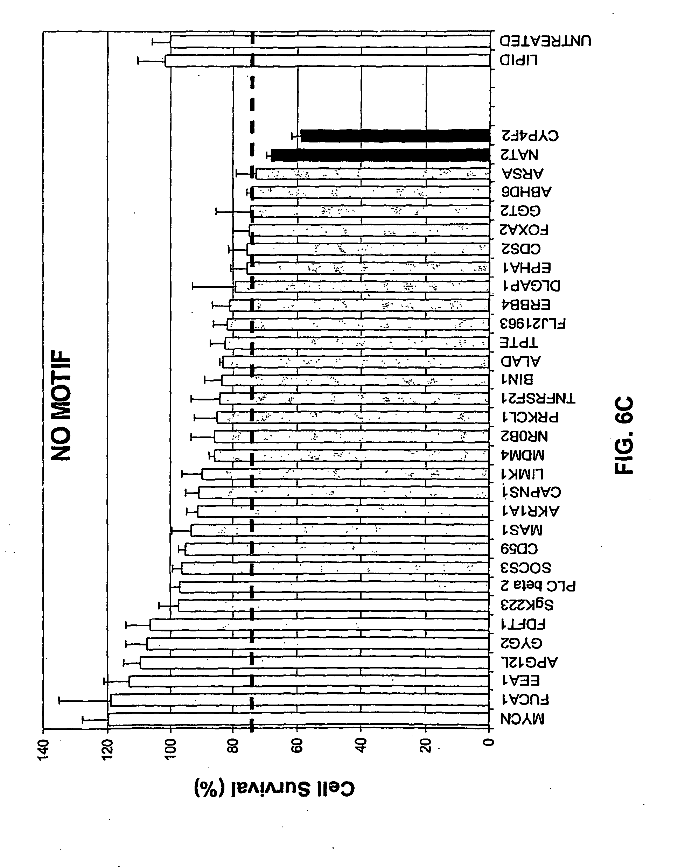

Figures 6A-6C are graphical representations of an embodiment of the identification of toxic motifs responsible for siRNA induced cell toxicity. Figure 6A depicts thirty-eight randomly selected siRNA containing AAA/UUU motifs tested for the ability to induce toxicity. Figure 6B depicts nineteen randomly selected siRNA carrying the GCCA/UGGC motif tested for the ability to induce toxicity. Figure 6C depicts thirty-two randomly selected siRNA that do not carry either the AAA/UUU or GCCA/UGGC motifs were tested for toxicity. In the figures, black bars represent sequences that induce toxicity, and gray bars represent non-toxic sequences.

Figure 7A is a schematic representation of embodiments of assays to study the involvement of the RNAi pathway in siRNA induced toxicity, which shows control and experimental studies.

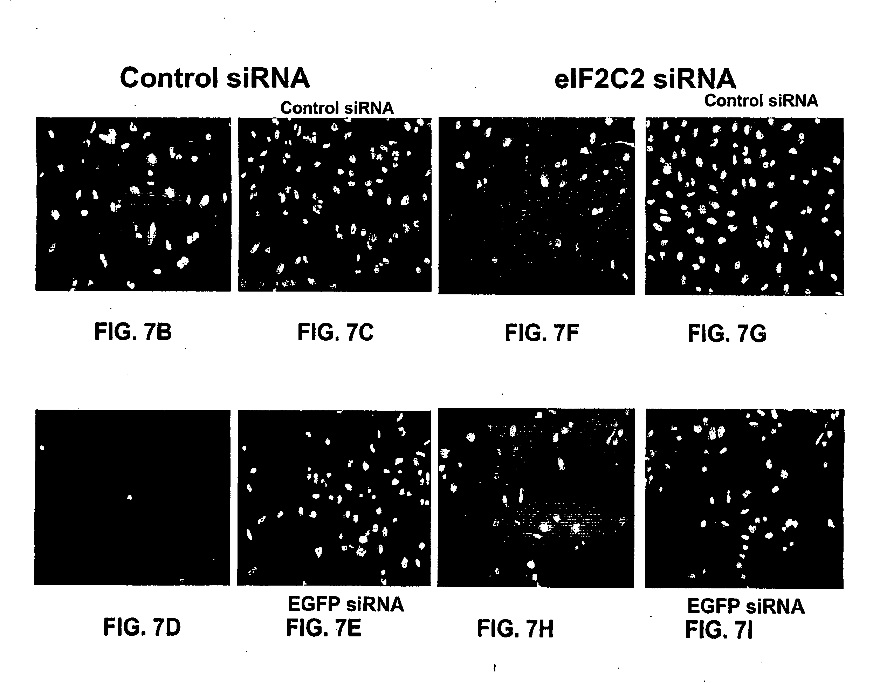

Figures 7B-7I are images of embodiments of green protein fluorescence resulting from the control and experimental conditions demonstrating that Ago2 silencing prevents siRNA targeting EGFP from silencing the intended target. Figure 7B depicts EGFP expression pattern and Figure 7C depicts Hoechst 33342 stained cells to show that treatment of cells with a control siRNA (*e.g.*, siRNA-RISC-Free) in both transfection 1 (T1) and transfection 2 (T2) does not affect EGFP expression. Figures 7D and 7E show when T1 is a control siRNA and T2 is an siRNA directed against EGFP, it is possible to silence EGFP expression. Figures 7F and 7G show when T1 uses an siRNA directed against the eIF2C2 protein (*e.g.*, Ago2), but T2 uses an control siRNA, EGFP expression is maintained. Figures 7H and 7I show when T1 uses an siRNA directed against the eIF2C2 protein, but T2 uses an EGFP-siRNA, EGFP expression is not affected due to the disruption of the RNAi pathway.

Figure 7J is a graphical representation of an embodiment of testing toxic siRNA in cells that have an intact RNAi pathway with control siRNA and a disrupted RNAi pathway with siRNA silencing eIF2C2/Ago2.

Figure 8 is a graphical representation of an embodiment of the effects of truncating toxic siRNA on cell viability.

Figure 9A is a graphical representation of an embodiment of the cell viability of OLIGOFECTAMINE™, Dharma*FECT*™ 1 ("DF1"), and TBio in A549 Cells.

Figure 9B is a graphical representation of an embodiment of the gene silencing of the conditions of Figure 9A.

Figure 10A is a graphical representation of an embodiment of the cell viability of OLIGOFECTAMINE™, Dharma*FECT*™ 1 ("DF1"), and TBio in 3T3L1 Cells.

Figure 10B is a graphical representation of an embodiment of the gene silencing of the conditions of Figure 10A.

Figures 11A and 11B are graphical representations of an embodiment of the gene silencing (Figure 11 A) and cell viability (Figure 11B) using three different media/buffer rehydration solutions, Opti-MEM™, HyQ-MEM™, and HBSS, in a reverse transfection format using Dharma*FECT*™ 1 ("DF1"). The numbers " 1 ", "2", "3", "4", "5" and "8" refer to various biological replicates performed on different days in this study.

## DETAILED DESCRIPTION OF THE PREFERRED EMBODIMENTS

Generally, the present invention is related to an apparatus and system for use in testing the efficacy of gene silencing in cells. The apparatus includes plates with wells that have dry control compositions comprised of control siRNA, which can be solubilized or suspended for use in RTF testing protocols. The systems, which can be provided as kits, include the plates and polynucleotide carriers that can be combined with the control siRNA to form a transfection complex capable of entering a cell in order to deliver the control siRNA. Additionally, the system or kits can include various other solutions and reagents for implementing RTF protocols.

The well plates, systems, kits, and methods of the present invention can be configured for use in high content screening ("HCS") and high throughput screening ("HTS") applications with or without the use of laboratory automation equipment. Also, the well plates, systems, kits, and methods can also be used with automated systems, such as robotic systems. However, the well plates, systems, kits, and methods can also be used in RTF testing protocols without the aid of automated delivery systems, or robotics, and thus can provide an efficient alternative to costly robotic delivery systems for laboratories using manual processing. Thus, the well plates, systems, kits, and methods provide versatility in choice such that high throughput screening can be done in a cost effective manner, wherein the efficacy of the gene silencing used in the screening can be tested along with the test siRNA.

The following terminology is defined herein to clarify the terms used in describing embodiments of the present invention and is not intended to be limiting. As such, the following terminology is provided to supplement the understanding of such terms by one of ordinary skill in the relevant art.

As used herein, the term "2' modification" is meant to refer to a chemical modification of a nucleotide that occurs at the second position atom. As such, the 2' modification can include the conjugation of a chemical modification group to the 2' carbon of the ribose ring of a nucleotide, or a nucleotide within an oligonucleotide or polynucleotide. Thus, a 2' modification occurs at the 2' position atom of a nucleotide. Examples of a 2' modification can include a 2'-O-aliphatic, 2'-O-alkyl, 2'-O-methyl, 2'-O-ethyl, 2'-O-propyl, 2'-O-isopropyl, 2'-O-butyl, 2'-O-isobutyl, 2'-O-ethyl-O-methyl (i.e., -$CH_2CH_2OCH_3$), 2'-O-ethyl-OH (i.e., -$OCH_2CH_2OH$), 2'-orthoester, 2'-ACE group orthoester, 2'-halogen, and the like.

As used herein, the term "antisense strand" is meant to refer to a polynucleotide or region of a polynucleotide that is at least substantially (*e.g.*, 80% or more) or 100% complementary to a target nucleic acid of interest. Also, the antisense strand of a dsRNA is complementary to its sense strand. An antisense strand may be comprised of a polynucleotide region that is RNA, DNA, or chimeric RNA/DNA. Additionally, any nucleotide within an antisense strand can be modified by including substituents coupled thereto, such as in a 2' modification. The antisense strand can be modified with a diverse group of small molecules and/or conjugates. For example, an antisense strand may be complementary, in whole or in part, to a molecule of messenger RNA ("mRNA"), an RNA sequence that is not mRNA (*e.g.*, tRNA, rRNA, and the like), or a sequence of DNA that is either coding or non-coding. The antisense strand includes the antisense region of polynucleotides that are formed from two separate strands, as well as unimolecular siRNAs that are capable of forming hairpin structures with complementary base pairs. The terms "antisense strand" and "antisense region" are intended to be equivalent and are used interchangeably.

As used herein, the terms "complementary" and "complementarity" are meant to refer to the ability of polynucleotides to form base pairs with one another. Base pairs are typically formed by hydrogen bonds between nucleotide units in anti-parallel polynucleotide strands. Complementary polynucleotide strands can base pair in the Watson-Crick manner (*e.g.*, A to T, A to U, C to G), or in any other manner that allows for the formation of duplexes. As persons skilled in the art are aware, when using RNA as opposed to DNA, uracil rather than thymine is the base that is considered to be complementary to adenosine.

Perfect complementarity or 100% complementarity refers to the situation in which each nucleotide unit of one polynucleotide strand can hydrogen bond with a nucleotide unit of an anti-parallel polynucleotide strand. Less than perfect complementarity refers to the situation in which some, but not all, nucleotide units of two strands can hydrogen bond with each other. For example, for two 20-mers, if only two base pairs on each strand can hydrogen bond with each other, the polynucleotide strands exhibit 10% complementarity. In the same example, if 18 base pairs on each strand can hydrogen bond with each other, the polynucleotide strands exhibit 90% complementarity. "Substantial complementarity" refers to polynucleotide strands exhibiting 79% or greater complementarity, excluding regions of the polynucleotide strands, such as overhangs, that are selected so as to be non-complementary. Accordingly, complementarity does not consider overhangs that are selected so as not to be similar or complementary to the nucleotides on the anti-parallel strand.

As used herein, the term "conjugate" is meant to refer to a molecule, large molecule, or macromolecular structure that is coupled with either the sense strand or antisense strand of an siRNA. That is, the moiety coupled to the siRNA is considered the conjugate. For clarity purposes, the siRNA can include a conjugate that is coupled thereto by a covalent bond, ionic interaction, and like couplings. Usually, a conjugate is coupled with an siRNA in order to impart a functionality other than increasing the stabilization or targeting specificity. For examples, some conjugates, such as cholesterol, can be used to enhance the ability of the siRNA to enter a cell. Other conjugates can be labels that can be used to detect transfection or the presence of the siRNA in the cell. Usually, the conjugate is coupled to the siRNA through a linker group.

As used herein, the term "control siRNA" is meant to refer to a type of siRNA that is well characterized, and can be used to test the efficacy of gene silencing that can be obtained from an RTF protocol. As such, a control siRNA can be used alone or with test siRNA and can provide results that can be compared to known and established results that have been standardized for that control siRNA. A control siRNA can be characterized as a positive control, negative control, and/or a transfection control. Control siRNA can be used to determine the efficacy of gene silencing that is being studied with a test siRNA. Control siRNA can be distinguished from test siRNA in that the control siRNA are well known and produce reproducable results, and are used as a control in a study that tests the ability of test siRNA to perform a gene silencing function, whereas test siRNA can have known or novel sequences and are usually tested for functionality against a target gene. Control siRNA are described in more detail below.

As used herein, the terms "dried" or "dry" as used in connection with gene silencing compositions is meant to refer to a composition that is not fluidic and does not flow. However, this does not exclude small amounts of water or other solvents, and includes amounts of water remaining in an RNA preparation that has equilibrated at standard or ambient conditions, for example, at one atmosphere of pressure, room temperature, and ambient humidity, such that the preparation is not in a substantially liquid form but instead is "dried" in the well. For example, an siRNA preparation is "dried" or substantially "dry" if, at about one atmosphere pressure, at about 20 to 40 °C, and at about 50 to about 95% humidity, the preparation is equilibrated and, when the well plate is inverted or tilted to, for example, 90° from horizontal, the RNA preparation does not displace or flow within the well. This is in comparison to a liquid preparation which would flow or run when tilted. In various embodiments, methods for using the dry gene silencing composition in order to perform a transfection can include solubilizing or suspending the dried preparation in a suitable aqueous medium to form a mixture. Additionally, the suitable aqueous medium can include a polynucleotide carrier capable of facilitating introduction of the siRNA into a cell, and exposing the mixture to one or more cells to achieve transfection.

As used herein, the term "duplex region" is meant to refer to the region in two complementary or substantially complementary polynucleotides that form base pairs with one another, either by Watson-Crick base pairing or any other manner that allows for a stabilized duplex between the polynucleotide strands. For example, a polynucleotide strand having 21 nucleotide units can base pair with another polynucleotide of 21 nucleotide units, yet only 19 bases on each strand are complementary such that the "duplex region" has 19 base pairs. The remaining bases may, for example, exist as 5' and/or 3' overhangs. Further, within the duplex region, 100% complementarity is not required, and substantial complementarity is allowable within a duplex region. Substantial complementarity refers to 79% or greater complementarity and can result from mismatches and/or bulges. For example, a single mismatch in a duplex region consisting of 19 base pairs results in 94.7% complementarity, rendering the duplex region substantially complementary.

As used herein, the term "functionality" is meant to refer to the level of gene specific silencing induced by an siRNA. In general, functionality is expressed in terms of percentages of gene silencing. Thus, 90% silencing of a gene (*e.g.*, F90) refers to situations in which only 10% of the normal levels of gene expression are observed. Similarly, 80% silencing of a gene (*e.g.*, F80) refers to situations in which only 20% of the normal levels of gene expression are observed.

As used herein, the term "gene silencing" is meant to refer to a process by which the expression of a specific gene product is inhibited by being lessened, attenuated, and/or terminated. Gene silencing can take place by a variety of pathways. In one instance, gene silencing can refer to a decrease in gene product expression that results from the RNAi pathway, wherein an siRNA acts in concert with host proteins (*e.g.*, RISC) to degrade mRNA in a sequence-dependent manner. Alternatively, gene silencing can refer to a decrease in gene product expression that results from siRNA mediated translation inhibition. In still another alternative, gene silencing can refer to a decrease in gene product expression that results from siRNA mediated transcription inhibition. The level of gene silencing can be measured by a variety of methods, which can include measurement of transcript levels by Northern Blot Analysis, B-DNA techniques, transcription-sensitive reporter constructs, expression profiling (*e.g.*, DNA chips), and related technologies and assays. Alternatively, the level of gene silencing can be measured by assessing the level of the protein encoded by a specific gene that is translated from the corresponding mRNA. This can be accomplished by performing a number of studies including Western Blot analysis, measuring the levels of expression of a reporter protein, such as colorimetric or fluorescent properties (*e.g.*, GFP), enzymatic activity (*e.g.*, alkaline phosphatases), or other well known analytical procedures.

As used herein, the term "nucleotide" is meant to refer to a ribonucleotide, a deoxyribonucleotide, or modified form thereof, as well as an analog thereof. Nucleotides include species that comprise purines, *e.g.*, adenine, hypoxanthine, guanine, and their derivatives and analogs, as well as pyrimidines, *e.g.*, cytosine, uracil, thymine, and their derivatives and analogs. Nucleotide analogs include nucleotides having modifications in the chemical structure of the base, sugar and/or phosphate, including, but not limited to, 5'-position pyrimidine modifications, 8'-position purine modifications, modifications at cytosine exocyclic amines, and 2'-position sugar modifications (*e.g.*, 2' modifications). Such modifications include sugar-modified ribonucleotides in which the 2'-OH is replaced by a group such as an H, OR, R, halo, SH, SR, $NH_2$, NHR, $NR_2$, or CN, wherein R is an alkyl or aliphatic moiety. Nucleotides are well known in the art. Also, reference to a first nucleotide or nucleotide at a first position refers to the nucleotide at the 5'-most position of a duplex region, and the second nucleotide is the next nucleotide toward the 3' end. In instances the duplex region extends to the end of the siRNA, the 5' terminal nucleotide can be the first nucleotide.

As used herein, the term "polynucleotide" is meant to refer to polymers of nucleotides linked together through internucleotide linkages. Also, a polynucleotide includes DNA, RNA, DNA/RNA, hybrids including polynucleotide chains of regularly and/or irregularly alternating deoxyribosyl moieties and ribosyl moieties (*i.e.*, wherein alternate nucleotide units have an -OH, then and -H, then an -OH, then an -H, and so on at the 2' position of a sugar moiety), and modifications of these kinds of polynucleotides. Also, polynucleotides include nucleotides with various modifications or having attachments of various entities or moieties to the nucleotide units at any position.

As used herein, the terms "rational design" and "rationally designed" are meant to refer to the selection or design of one or more siRNA(s) for use in a gene silencing application based upon one or more criteria that are independent of the target sequence. As such, rationally designed siRNA are selected to specifically interact with and inhibit polypeptide translation from a selected mRNA. Thus, for any one target mRNA there may be hundreds of potential siRNA having from 18 to 31 base pairs that are 100% complementary to the target mRNA. In part, this is because a single mRNA may have multiple sequences that can be specifically targeted by the siRNA. However, it is likely that not all of the siRNA will have equal functionality. Through empirical studies, a number of other factors including the presence or absence of certain nitrogenous bases at certain positions, the relative GC content, and the like, can affect the functionality of particular siRNA. Additional information regarding rationally designed siRNA can be found in commonly owned U.S. Patent Application, 10/714,333, filed on November 14, 2003 and published as US-2007-0031844, related PCT application PCT/US03/36787, published on June 3, 2004 as WO 2004/045543 A2, U.S. Patent Application 10/940,892, filed on September 14, 2004, published as U.S. Patent Application Publication 2005/0255487, related PCT application PCT/US 04/14885, filed on May 12, 2004, and U.S. Patent Application Publication 2005/0246794.

As used herein, the term "reverse transfection" and abbreviation "RTF" are each meant to refer to a process for introducing nucleic acid, such as an siRNA, into a cell. Such an introduction of an siRNA into a cell can be accomplished by combining the nucleic acid and cell in a well, wherein the cell has not yet been previously adhered or maintained on the growth surface. The reverse transfection proceeds by contacting the nucleic acid onto a cellular surface in a manner such that the nucleic acid can enter into the cell. Usually, the siRNA is complexed with a lipid or other polynucleotide carrier prior to being contacted to the cells. Reverse transfection differs from forward transfection because the cells have not been speeded and maintained on the cellular growth surface of a well or other container before addition of the siRNA.

As used herein, the term "sense strand" is meant to refer to a polynucleotide or region that has the same nucleotide sequence, in whole or in part, as a target nucleic acid such as a messenger RNA or a sequence of DNA. The term "sense strand" includes the sense region of a polynucleotide that forms a duplex with an antisense region of another polynucleotide. Also, a sense strand can be a first polynucleotide sequence that forms a duplex with a second polynucleotide sequence on the same unimolecular polynucleotide that includes both the first and second polynucleotide sequences. As such, a sense strand can include one portion of a unimolecular siRNA that is capable of forming hairpin structure, such as an shRNA. When a sequence is provided, by convention, unless otherwise indicated, it is the sense strand or region, and the presence of the complementary antisense strand or region is implicit. The phrases "sense strand" and "sense region" are intended to be equivalent and are used interchangeably.

As used herein, the term "siRNA" is meant to refer to a small inhibitory RNA duplex that induces gene silencing by operating within the RNA interference ("RNAi") pathway. These siRNA are dsRNA that can vary in length, and can contain varying degrees of complementarity between the antisense and sense strands, and between the antisense strand and the target sequence. Each siRNA can include between 17 and 31 base pairs, more preferably between 18 and 26 base pairs, and most preferably 19 and 21 base pairs. Some, but not all, siRNA have unpaired overhanging nucleotides on the 5' and/or 3' end of the sense strand and/or the antisense strand. Additionally, the term "siRNA" includes duplexes of two separate strands, as well as single strands that can form hairpin structures comprising a duplex region, which may be referred to as short hairpin RNA ("shRNA").

As used herein, the terms "siRNA pool," "pool," "pool of siRNAs," and "pool reagents" are meant to refer to two or more siRNA, typically four siRNA, directed against a single target gene, mRNA, and/or translation of a protein. The siRNA of the pool reagent can be rationally designed by being selected according to non-target specific criteria as described herein and in the references cited herein. For example, two nanomoles of each pool reagent can be sufficient for transfecting cells in about 200 wells of multiple 96-well plates, using 100 nM siRNA concentration. Pool reagents can be plated as a pool (*i.e.*, the two or more siRNA of Dharmacon's *SMART*pool® Reagent in a single transfection well). The individual siRNAs that comprise the *SMART*pool® Reagent can also be plated individually on the same plate as the *SMART*pool® Reagent.

As used herein, the term "target" is used in a variety of different forms throughout this document and is defined by the context in which it is used. The term "target gene" is meant to refer to the gene that encodes the protein to be silenced by the siRNA, and encodes for the production of the target mRNA. The term "target mRNA" is meant to refer to an mRNA against which a given siRNA is direct to silence the transcription of the polypeptide product. The term "target sequence" and "target site" are meant to refer to a sequence within the mRNA, miRNA, or DNA coding or promoter region to which the sense strand of an siRNA exhibits varying degrees of homology and the antisense strand exhibits varying degrees of complementarity. The term "target polypeptide" or "target protein" is meant to refer to the gene product encoded by the target gene, target mRNA, and/or target sequence. The term "siRNA target" can refer to the gene, mRNA, or protein against which the siRNA is directed to for silencing. Similarly, "target silencing" can refer to the state of silencing a gene, or the corresponding mRNA or protein.

As used herein, the term "transfection" is meant to refer to a process by which nucleic acids are introduced into a cell. The list of nucleic acids that can be transfected is large and includes, but is not limited to, siRNA, shRNA, sense and/or anti-sense sequences, DNA, RNA, and the like. There are multiple modes for transfecting nucleic acids into a cell including, but not limited to, electroporation, calcium phosphate delivery, DEAE-dextran delivery, lipid delivery, polymer delivery, molecular conjugate delivery (*e.g.*, polylysine-DNA or -RNA conjugates, antibody-polypeptide conjugates, antibody-polymer conjugates, cholesterol conjugates, or peptide conjugates), microinjection, laser- or light-assisted microinjection, optoporation or photoporation with visible and/or nonvisible wavelengths of electromagnetic radiation, and the like. Transfections can be "forward transfections" whereby cells are first plated in wells and then treated with a nucleic acid or they can be "reverse transfections" (RTF) whereby the nucleic acid is combined with the cells before or during being plated and/or attached to the bottom of the well. Any mode of transfecting cells, such as those described above, can be used with the present invention by inducing the nucleic acid to be introduced into a cell after the siRNA is solubilized or suspended in the aqueous medium to implement reverse transfection. Details regarding a mode of reverse transfection are described in more detail below

As used herein, the term "well plate" is meant to refer to a substrate that is divided into distinct regions that prevent migration from one distinct region to another distinct region, wherein the distinct regions are wells. For example, each well of a multi-well well plate may contain a horizontal well floor that may be curved or flat, as well as have sidewalls. Additionally, well plates are well known in the art.

The use of units to define measurable quantities of material, such as concentration, weight, and volume, are intended to be those that are routinely employed by those of skill in the art. Additionally, the units are preferably interpreted to correspond with the metric system. Also, the use of "u," as in "ug" or "uL" is meant to refer to "micro" as applied to microgram and microliter, respectively.

Additionally, while the foregoing term definitions are intended to supplement the knowledge of one of ordinary skill in the art, not every term within this document has been defined. As such, the undefined terms are intended to be construed with the knowledge of one of ordinary skill in the art and/or the plain meaning of the term. Additionally, the foregoing terms are not intended to be limited by the examples provided therein, but are intended to be useful in understanding and practicing the invention as described herein.

## I. Reverse Transfection

Generally, the present invention provides well plates, systems, kits, and methods for testing and/or optimizing the efficacy of procedures and/or conditions that implement reverse transfection ("RTF") of siRNA. As such, the present invention can provide for plates, systems, kits, and methods that can be used to assess or test the efficacy of gene silencing. The present invention provides methods of determining or assessing the efficacy of gene silencing so that improvements in both reverse transfection methodologies that pertain to siRNA and the efficiency of siRNA based gene silencing can be obtained. In part, this is because the plates, systems, kits, and methods use control siRNA that can provide meaningful results pertaining to the accuracy of results obtained from a gene silencing protocol. Thus, the results obtained from using control siRNA can be used to validate or invalidate gene silencing results, and lead to the improvement of siRNA RTF protocols and the conditions used therewith.

In one embodiment, the present invention includes a method of testing and/or optimizing the effectiveness of an siRNA RTF protocol and/or condition for introducing siRNA into a cell to effect gene silencing. Such a method can include providing a well plate that includes a well having a substantially dry control composition. The control composition can include an individual control siRNA or a pool of control siRNAs. The control composition can include a control siRNA which can be used to provide meaningful information regarding the effectiveness of the siRNA RTF protocol and/or condition. Such meaningful information can provide an indication of whether or not the RTF protocol and/or condition is sufficient for introducing test siRNA into cells to effect gene silencing. Also, the meaningful information can provide an indication of whether or not any gene silencing data obtained from the siRNA RTF protocol is valid and reliable, or whether the data inaccurately represents the effectiveness of a test siRNA to silence a target gene. Also, the control siRNA can provide an indication of whether any gene silencing is a function of cellular toxicity or non-specific gene silencing rather than that resulting from specific gene silencing. Moreover, the method of testing the effectiveness of an siRNA RTF protocol and/or condition can be used as part of an optimization procedure so that the optimum gene silencing conditions can be selected for certain siRNA, cells, polynucleotide carriers, and the like.

The control siRNA can be present in the well of a plate as part of a dry control composition so that the test plates or optimization plates can be prepared, sealed, stored, and/or shipped long before an RTF testing protocol is performed. In part, this is because the dry control composition can stably retain the control siRNA in a usable condition within the well, and be resuspended or resolubilized with an aqueous medium during the RTF testing protocol. Thus, a well plate having the control composition can be manufactured and hermetically sealed in an inert environment within a sterile package, wherein the plate can include different wells with predefined types of control siRNA and optionally test siRNA for specific gene targets. Such types of control siRNA intended to test the effectiveness of an siRNA RTF protocol are described in more detail below.

In any event, the testing can be performed by adding an aqueous medium to each well that contains a control composition so as to suspend or solubilize the control siRNA into the aqueous medium. The aqueous medium is allowed to solubilize or suspend the control siRNA for a sufficient duration so that most, if not all, of the control siRNA is solubilized or suspended. Optionally, the aqueous medium or an additional solution is comprised of a polynucleotide carrier. However, polynucleotide carriers are not necessary in some embodiments.

After the control siRNA is adequately solubilized or suspended, cells are added to the well under conditions that permit the control siRNA to be introduced into the cell. The cells can be added in an amount of about $1 \times 10^3$ to about $3.5 \times 10^4$ cells per about 0.3 cm$^2$ to about 0.35 cm$^2$ of cell growth surface area. The conditions that promote the control siRNA entering the cell can be described by typical cell culture techniques used for plating cells that are well known in the art, and further can be the conditions used in an siRNA RTF protocol to be tested. That is, the cells can be added to the well that contains the control siRNA in a manner similar to ordinary plating. Optionally, the cells are added to a well having a dry control composition so that the aqueous medium carrying the cells can solubilize or suspend the control siRNA. The well containing the control siRNA and cells can be incubated for a sufficient duration for gene silencing to occur, which is typically less than 72 hours, more preferably less than 48 hours, and most preferably about 24 hours or less.

In one embodiment, the methods include transfecting the cells with the siRNA. As such, any mode of transfection can be implemented in the RTF format by adding the cells to the well having the siRNA. Accordingly, the cells can be transfected while suspended or while attaching to the well floor. The modes of transfection can include those described above or others known or developed later.

In one embodiment, the RTF testing protocol can include adding the polynucleotide carrier to the well so as to form a control complex, wherein the control complex is suspended or solubilized in the aqueous medium. After the cells are added, the control complex can be contacted to the cell to induce endocytosis of the complex. As such, the polynucleotide carrier can be added as part of the aqueous medium or in addition thereto. Thus, the polynucleotide carrier can be presented in an aqueous medium and be either solubilized or suspended therein. The polynucleotide carrier can be a cationic lipid, polymer, lipopolymer, and the like.

After the cells are combined with the control siRNA, the well plate can be maintained under conditions so that cell growth, cell division, transfection, and/or gene silencing occurs. Usually, the cells are maintained in the presence of the control siRNA for about 6 to about 72 hours before gene silencing is assessed, more preferably about 12 to about 36 hours, and most preferably for about 24 to about 48 hours. However, it should be recognized that the cells are incubated with the control siRNA for a time period sufficient for silencing a gene so that the amount corresponding gene product decreases. As such, the production of a target polypeptide can be silenced by at least about 50%, more preferably by at least about 70%, even more preferably by at least about 80%, and most preferably by at least about 90%.

In instances where cells that grow in suspension are the target cell, such cells can be added to the wells at an appropriate cell density and plates can be spun under low gravity forces that are not detrimental to cell viability to bring the cells and lipids into close proximity on the bottom of the well.

In one embodiment, the control composition includes a positive control siRNA. The positive control siRNA can be characterized by being capable of silencing expression of a known gene. Also, the positive control siRNA can provide consistent, reproducible, and known results that can be used as assess the gene silencing efficacy of an RTF protocol and/or condition. For example, the positive control siRNA can silence at least one of a MAP kinase gene, cyclophilin B gene, lamin A/C gene, glyceraldehyde-3-phosphate dehydrogenase gene or other well-known and established control genes that can be reproducibly silenced

In one embodiment, the control composition includes a transfection control siRNA. The transfection control siRNA can provide the ability to assess the level or percentage of cells that have been successfully transfected with the transfection control siRNA. That is, a transfection control siRNA can be configured to identify whether or not it has successfully entered into a cell. In one aspect, the transfection control siRNA includes a label that can be detected in a cell. This allows the cells to be analyzed to determine whether or not the transfection control siRNA is present in the cell. Examples of labels include colorimetric labels, chemiluminescent labels, fluorescent labels, mass labels, and radioactive labels. The labels can be considered conjugates and can be coupled with siRNA as described for conjugates herein and in the references cited herein. This includes a direct coupling to the siRNA and coupling through a linker. The label can be coupled to a 5' terminal nucleotide or 3' terminal nucleotide on one of a sense strand or an antisense strand. Optionally, the label can be attached to any nucleotide on the sense strand or antisense strand. However, it is preferable for the label to be fluorescent, and be coupled to the sense strand.

In one embodiment, the transfection control siRNA can be toxic to cells. This can include siRNA that include at least one toxic motif. That is, the siRNA includes a polynucleotide sequence that can lead to cellular toxicity and/or cell death. Examples of such toxic motifs can include siRNA that include a polynucleotide sequence including an AAA motif, UUU motif, GCCA motif, or UGGC motif. However, other toxic siRNA can be employed as transfection control siRNA.

In one embodiment, the control composition includes negative control siRNA. The negative control siRNA can be configured to be non-functional siRNA. That is, the siRNA can include a polynucleotide sequence that does not function in the RNAi pathway. For example, the negative control siRNA can have a polynucleotide sequence that does not target any known human gene, animal gene, or gene within the cell being transfected and studied. Also, a non-functional siRNA can include a 17 base pair duplex containing a sense strand with 2' modifications at the first and second nucleotide, and an antisense strand with 2' modifications at the first and second nucleotide. Alternatively, the non-functional siRNA can include a 19 base pair duplex having 5' deoxy nucleotides on the 5' terminal nucleotides of the sense and/or antisense strands.

In one embodiment, the negative control siRNA can be configured to inhibit RISC uptake and processing. This can include a chemically modified siRNA that inhibits being taken in or processed by RISC.

In one embodiment, the cells transfected with the control siRNA during the RTF testing protocol can be assessed for cell viability, control gene silencing, control polypeptide production, control mRNA amount, presence of the control siRNA, non-functionality of the control siRNA, and the like. The cell viability studies can be performed in the well plate in accordance with well known procedures. Additionally, the control gene silencing can also be assessed with the contents in the well by various techniques well known in the art to assess the presence or absence of target proteins. When the control siRNA includes a label, it can be detected in the cell. Alternatively, the amount of gene silencing can be assessed by removing the contents from the well by well known assays. In various embodiments, the well is designed to be compatible with optical detection systems such as, for example, UV, luminescence, fluorescence, or light scattering detection systems, which can be favorable for transfection control siRNA having fluorescent labels. In embodiments compatible with optical detection systems, the walls of the well can be made opaque, or rendered such that light scattering that can interfere with optical detection is reduced or minimized.

In one embodiment, the results of the RTF protocol to induce gene silencing can be detected or monitored using systems for performing high content screening ("HCS") or high throughput screening ("HTS"). An HCS analysis can be used to measure specific translocation and morphology changes, receptor trafficking, cytotoxicity, cell mobility, cell spreading, and the like. HCS studies can be performed on an ArrayScan® HCS Reader, or a KineticScan® HCS Reader (Cellomics, Inc.) Additional information on HCS can be found in U.S. Patent Nos. 6,902,883, 6,875,578, 6,759,206, 6,716,588, 6,671,624, 6,620,591, 6,573,039, 6,416,959, 5,989,835. HTS analyses can be performed using a variety of available readers, typically of the fluorescence from each well as a single measurement.

In one embodiment, the invention includes a well plate configured for having the contents of a well transferred to a location, device, or system wherein detection of the results of an siRNA RTF testing protocol is carried out. As such, wet transfer detection systems can be employed that include systems wherein cells are transferred from wells to a substrate such as nitrocellulose. Following the transfer of the well contents to the substrate a detection protocol can be implemented. An example of such a well plate transfer system can include nitrocellulose, wherein the well contents can be treated such that cell membranes are permeabilized or disrupted so as to gain access to intracellular contents. The transfer of the well contents to the nitrocellulose can be achieved by any suitable method including gravity or use of a vacuum manifold. The nitrocellulose containing the well contents can then be further subjected to a detection protocol that uses antibody-based detection systems and the like to detect the presence or level of one or more contents of the cells that comprise a particular well.

## II. Optimizing siRNA RTF

Due to the unique and highly sensitive nature of the RNAi pathway, methodologies particularly useful for introducing siRNA into cells have been developed. Recently developed protocols for implementing siRNA RTF were modified by augmenting such protocols with recently developed siRNA technologies based on rationale design, siRNA stabilization, siRNA targeting specificity, and pooling siRNAs. However, the effectiveness of new siRNA RTF protocols can still be compromised by a lack of optimization, errors, or improper conditions. Thus, methods for testing and optimizing the efficacy of gene silencing with siRNA RTF formats are presented herein.

It has recently been discovered that there are many diverse responses when different cell numbers, different lipids, and different lipid to siRNA ratios are used in an RTF format in comparison with those that are recommended for forward transfection protocols. Accordingly, control siRNA can be used in RTF testing protocols to assess the conditions in which a test siRNA is employed.

The number of cells per well, which is referred to as the cell density, is an important parameter of successful siRNA pool RTF. It has been found that siRNA pool RTF protocols can have more favorable results with lower cell densities compared to RTF protocols using DNA. For example, 96-well plates can include cell densities of about 1,000-35,000 cells per well, more preferably about 1,250-30,000 cells per well, even more preferred are cell densities of about 1,500-20,000 cells per well, still more preferably about 1,750-15,000 cells per well, and most preferable are cell densities of about 3,000-10,000 cells per well. Also, the number of cells per well can be extrapolated to wells having different cell culture areas. One possible equation for calculating the appropriate number of cells that are placed in a given well is based on a 96-well plate having a cell culture area of about 0.3 cm$^2$ to about 0.35 cm$^2$, wherein well # 2 is the 96-well plate, and is described as follows:

$$\text{cells in well \# 1} = \left( \frac{\text{area of well \#1}}{\text{area of well \#2}} \right) \times \text{cells in well \#2}$$

Additionally, siRNA RTF testing protocols can be used to determine whether a particular polynucleotide carrier, such as a lipid, can be useful in a particular siRNA RTF condition. The polynucleotide carrier can be tested over a wide range of carrier concentrations by using a robust and easily-transfected cell line (*e.g.*, HeLa) with control siRNA over commonly used ranges of cell density and siRNA concentrations. Accordingly, cell viability and the function of the control siRNA can be assayed with the foregoing concentration gradients. Thus, optimization studies can be performed with concentration gradients in order to determine which polynucleotide carriers can produce highly efficient transfection without inducing unfavorable cell toxicity.

In one embodiment, the present invention is directed to optimization of siRNA RTF protocols for implementing gene silencing through the RNAi pathway. As such, optimization of siRNA RTF can include any of the following: (1) selecting the type of plate; (2) selecting an appropriate solution to solubilize or suspend the siRNA for being deposited and dried in a well; (3) selecting at least one control siRNA or a pool of control siRNAs, which can be a transfection control, positive control, or a negative control; (4) identifying any modifications or conjugates that can be applied to the individual siRNA in order to enhance siRNA stability and/or specificity; (5) applying and drying the siRNA on a solid surface so that it can be solubilized or suspended in an appropriate aqueous medium; (6) selecting an appropriate mode of transfection; (7) selecting a polynucleotide carrier for siRNA such as a lipid; (8) solubilizing or suspending an siRNA; (9) complexing the siRNA with the polynucleotide carrier to form an siRNA-carrier complex; and (10) combining the siRNA-carrier complex with the cell type or types of choice. Thus, optimizing siRNA RTF protocols can result in a dramatic improvement over previous forward and reverse transfection procedures.

In one embodiment, the present invention can include siRNA RTF test protocols to implement along with the foregoing optimizations, which can include any of the following: (a) applying at least one control siRNA to two or more wells of a multi-well plate; (b) drying the control siRNA on the floor of each well; (c) adding an aqueous medium such as a media or buffer to the control siRNA in each well in order to solubilize or suspend the control siRNA, and optionally the solution includes a polynucleotide carrier so that a control complex can form; (d) adding an appropriate number of cells to each well in which the control siRNA is already in solution alone or as an control complex; and (e) after cells have been added, maintaining the plate under conditions in which transfection of the cells by the control siRNA can occur. Following transfection, the cells are subjected to conditions, such as liquid media, temperature, gas partial pressures, and the like, in which cell growth and/or cell division will occur and gene silencing may occur. These conditions can be, but not necessarily, the same as the conditions under which transfection occurs, and are well known in the art.

### III. Well Plates

In one embodiment, the present invention includes the use of control solutions dried in the bottom of a well in a well plate. The well plates used in connection with the present invention are preferably formatted and distinct well arrays (*e.g.*, a 48, 96, 384, or 1536 well plate) that can be purchased from any number of commercial sources of cell culture plates and other cell culture surface-containing devices.

A well plate can be made of glass, polystyrene, other polymeric material or any equivalent materials, and can form a rounded or generally flat horizontal bottom having various generally planar shapes. Additionally, wells having substantially flat floors can provide uniform cell spacing and monolayer formation and are preferred. Additionally, it can be preferable for each plate to have between 32 and 2000 wells, and more preferably having 1536 wells, 384 wells, or 96 wells; however, a plate having any number of wells can be used. Also, it can be preferable for the wells to have a volume that varies between about 5 to about 200 microliters ("uL"), and the total culture area, which is represented by the well floor, to range between about 0.02 cm$^2$ and about 0.35 cm$^2$. Additionally, the wells may not be modified by any chemical coating, or they can be coated with poly-L-lysine ("PLL"), laminin, collagen, or equivalent substances that improve the adherence of cells. Additional descriptions of well plates can be reviewed in the cross-referenced application having Attorney Docket No. 16542.1.1 and Publication No: US-2006-0115461, entitled APPARATUS AND SYSTEM HAVING DRY GENE SILENCING COMPOSITIONS, with Barbara Robertson, Ph.D., et al. as inventors.

Additionally, any of the plates can be included in systems or kits in accordance with the present invention. Such kits can include the plates having control compositions and can be distributed with siRNA solubilizing or suspending solutions, polynucleotide carriers, carrier solutions, reagents, cell media, and the like.

### IV. Reverse Transfection Testing Plates

In one embodiment of the present invention, a well plate in accordance with the foregoing can be configured as a reverse transfection testing plate ("RTF testing plate"). Accordingly, the RTF testing plate can include a control composition in one or more wells. The control composition includes at least a first control siRNA that can provide an indication of the efficacy of gene silencing. Optionally, the control composition can have a pool of control siRNAs. Well plates can be RTF testing plates by having a control siRNA-containing solution applied to at least one well, which is then dried in a manner that removes the solution and leaves a dried control composition.

In some instances the control siRNA can be solubilized in one of several types of solutions prior to applying, depositing, and/or spotting the control siRNA solution onto the well floor, and drying the material on the plate. The control siRNA can be dissolved in distilled water that has been treated by one of any number of art-recognized techniques to eliminate contamination by RNases such as by ultrafiltration. Alternatively, the control siRNA may be dissolved in one of several physiologically compatible, RNase-free buffers, including but not limited to phosphate buffer, Hanks BSS, Earl's BSS, or physiological saline. These solutions may contain one or more additional reagents that enhance the stability of the control siRNA (*e.g.*, RNase inhibitors) or alter the viscosity of the solution to enhance spotting or drying efficiency (*e.g.*, sucrose) without changing the properties of the control siRNA or injuring the cells that are added at subsequent stages in the RTF testing protocol.

In still other cases, the control siRNA may be solubilized in a solution or medium that will enhance spotting, drying, or sticking to the plate of choice. Optionally, volatile solvents can be used that are compatible with control siRNA. One example includes the use of alcohols, such as ethanol, which can be mixed with water in order to form a volatile solvent that can be readily dried and leave a dry control composition on the well floor. In some instances the solution of control siRNA does not contain lipids that are easily oxidized over the course of time or can be toxic to cells. In other instances the control siRNA is pre-complexed with a polynucleotide carrier in a solution before being deposited and dried to the well floor.

Accordingly, a predefined amount of control siRNA can be administered to the well so that when it is dried and then resuspended, a known amount or concentration of control siRNA is available for testing gene silencing. The volume of solutions that are deposited on the bottom of each well can depend upon the concentration of the stock solution, functionality of the control siRNA, and desired amount or concentration of control siRNA available for testing gene silencing. For example, the concentration of siRNA during transfection can range from picomolar (*e.g.*, 300-900 pM) for highly functional siRNA (*e.g.*, silence >90% of target expression at 50-100 nM), to nanomolar (*e.g.*, 100 nM) for siRNA of intermediate functionality (*e.g.*, 70-90% silencing of target expression at 50-100 nM), and to micromolar (*e.g.*, 1 uM) for low functionality. For example, for a 96-well plate, deposition of 5-50 uL of a 1 uM siRNA-containing solution is sufficient to generate an acceptable concentration of control siRNA for RTF testing protocols. For smaller or larger sized wells, volumes and amounts of control siRNA would be adjusted to compensate for the final concentration of lipid-media/buffer and media that can be accommodated in each well.

In one embodiment, the total amount of siRNA in the control composition can be present in an amount for transfecting cells in only the well in which it is contained. As such, the total concentration of control siRNA can be less than about 100 nM when solubilized or suspended in the aqueous medium during RTF, more preferably less than about 50 nM, even more preferably the total concentration of siRNA can be less than about 25 nM, and most preferably less than about 10 nM when solubilized or suspended in the aqueous medium during RTF. In another option, the total concentration of control siRNA can be less than about 1 nM when solubilized or suspended in the aqueous medium during RTF. For example, the amount of control siRNA in a 96-well plate can be from 0.1 picomoles ("pm") to about 100 pm, more preferably about 1 pm to about 75 pm, and most preferably about 10 pm to about 62.5 pm per well, where corresponding amounts of control siRNA can be calculated for plates having other numbers of wells.

Additionally, the amount of control siRNA added to each well can be sufficient for use in a single RTF testing protocol within that well. That is, the control siRNA in the control composition can be present in an amount to only be used with the cells added to the well. As such, the amount of control siRNA dried in the well can be insufficient for performing two RTF protocols in two different wells. This is because the amount of control siRNA provided in the control composition is configured for a single RTF testing protocol in order to produce optimal results. Also, this eliminates the need to make a stock siRNA solution that is transferred into multiple wells, thereby reducing the complexity of the RTF testing protocol and increasing efficacy.

The control siRNA-containing solutions can be deposited into wells using various well known techniques in the art for depositing liquids into wells of well plates, which can include manual and automated processes. Various methods can be used to dry the control siRNA-containing solution into a control composition. In one embodiment, the plates are allowed to dry at room temperature in a sterile setting which allows the deposition solution to evaporate leaving behind the control siRNA and any other conditioning compounds, such as salts, sugars, and the like. Dried plates are preferably vacuum-sealed or sealed in the presence of inert gases within a sterile container, and stored at temperatures ranging from -80°C to 37°C for extended periods of time without loss of silencing functionality. Thus, the plates having the dry control compositions in at least one well can be stored at room temperature and shipped via traditional routes and still maintain the integrity and functionality of the control siRNA.

In one embodiment, the plate can further include a blank well devoid or substantially devoid of siRNA. That is, the blank well does not have enough siRNA to provide meaningful gene silencing. The blank well can be used in concert with control siRNA to show the effects of the gene silencing condition in the absence of any siRNA. This can be especially beneficial when assessing the toxicity of test or control siRNA, or measuring the amount of a particular mRNA or protein present in a cell. Also, this can provide data regarding the effect of the polynucleotide carrier on the cell in the absence of siRNA.

In one embodiment, the plate can further comprise at least one test well that has a substantially dry gene silencing composition. The gene silencing composition can have at least a first test siRNA which silences a test target gene. Additionally, the gene silencing composition can be configured such that the test siRNA is capable of being solubilized or suspended in an aqueous medium in an amount sufficient for transfecting cells in the well. The gene silencing composition can be characterized by at least one of the following: (a) the test siRNA can be unmodified; (b) the test siRNA can have a stabilizing modification;(c) the test siRNA can have a modification to limit off-targeting; (d) the test siRNA can have a conjugate; or (e) the test siRNA can have a hairpin structure.

## V. <u>Control siRNA</u>

In one embodiment, the dry control compositions include at least a first control siRNA which can provide an indication of the efficacy of gene silencing. Optionally, the dry control composition can include a pool of control siRNAs. The control composition is configured such that the control siRNA is capable of being solubilized or suspended in an aqueous medium in an amount sufficient for transfecting cells in the well. Optionally, the total amount of control siRNA in the well is sufficient for implementing reverse transfection only for that well. Additionally, it is optional for the control siRNA to have at least one of a modification or a conjugate. Also, the control siRNA can be rationally designed to target a control gene. Furthermore, the control composition can include a pool of control siRNAs. Examples of control siRNA include transfection control siRNA, positive control siRNA, and negative control siRNA.

The ability to use control siRNA in a control well to test the efficacy of gene silencing in other test wells is based on the consistency between RTF protocols and conditions. In part, consistency between conditions of a control well and a test well may enable the results of the control well to provide meaningful results with regard to the test well. Consistency between the control well and test well can include consistency in cell type, cell seeding number, cell density, mode of transfection, polynucleotide carrier type, polynucleotide carrier concentration, siRNA concentration, cell culture media, any environmental conditions to which the both wells are subjected, and the like. In the instance in which a number of factors are different between the control well and the test well, the results obtained from the control well may not be indicative of the gene silencing efficacy of the test siRNA. Thus, it can be beneficial for the control well and test well to be on the same plate, or run in concert with conditions and protocols that are as similar as possible.

Often, control siRNA are utilized to determine whether or not the protocol or conditions associated with RTF protocol can induce non-specific gene silencing or, in some instances, a unique phenotype. The results obtained from using control siRNA can then be compared to the test data obtained from test siRNA, wherein the test siRNA are the siRNA that are being studied in the gene silencing experiments. Additionally, the control siRNA can be characterized by the following: (a) the control siRNA can be unmodified; (b) the control siRNA can have a stabilizing modification; (c) the control siRNA can have a modification to limit off-targeting; (d) the control siRNA can have a conjugate; or (e) the control siRNA can have a hairpin structure.

**A. Transfection Control siRNA**

Transfection control siRNA are configured to enable the level or percentage of cells that have been transfected during an RTF testing protocol to be identified and/or quantified. This includes control siRNA that can be used to monitor the efficiency of an RTF procedure and/or condition. The level of transfection or percentage of cells that have been transfected can be measured by a variety of methods, which include identifying the presence of transfected control siRNA in a cell or measuring the effects of the control siRNA on the cell. When the effects of control siRNA are measured, the effects can be direct or indirect, but are usually well known and reproducible effects that are specifically caused by the control siRNA. In any event, transfection control siRNA are used to monitor the efficacy of an RTF protocol and/or condition by comparing the resulting effects in the control well with established and reproducible effects that correspond with the specific transfection control siRNA. This provides an indication of the transfection and/or gene silencing efficiency.

In the instance where the presence of the transfection control siRNA in a transfected cell is identified or quantified during an RTF testing protocol, the control siRNA can be a type that is detected without regard to any functionality. That is, the control siRNA can be configured to be detected in a cell without measuring any effect that may have been induced. Such transfection control siRNA can include a label that can be directly measured by techniques well known in the art. Examples of labels can include colorimetric labels, fluorescent labels, luminescent labels, chemiluminescent labels, enzymatic labels, mass labels, radioactive labels, and the like. Thus, the presence of the control siRNA in a cell can be measured within the contents of the cell being retained within the well, or the contents can be removed for analysis. Any untransfected siRNA can be removed from the cell culture by well known washing protocols.

In one embodiment, the transfection control siRNA includes a colorimetric label. A colorimetric label can be a chromophore that is detectible by measuring and analyzing the absorbance or transmitted color spectrum or single wavelength of a sample. As such a chromophore can be coupled to the siRNA so that the transfection can be detected or measured by the color spectrum or single wavelength that is transmitted in response to incident light or measuring the absorbance of the sample. This can also be compared to the color spectrum that can be obtained from cells in a blank well. Alternatively, a colorimetric label can be an enzyme that reacts with a specific substrate to generate a chromophore product. The label can be measured and/or quantified by measuring the amount of light absorbed or transmitted by the product at a specific wavelength or spectrum. For example, enzymes that can be used as labels include alkaline phosphatase with para-nitrophenyl phosphate as the substrate, horseradish peroxidase with hydrogen peroxide/coupler as the substrate, β-galactosidase with O-nitrophenylgalactoside as substrate and the like.

In one embodiment, the transfection control siRNA includes a fluorescent label. The fluorescent label can be used in order to photometrically monitor the delivery of the control siRNA into a cell. Preferably, the fluorescent label is a rhodamine or a fluorescin; however, other fluorescent molecules that can be coupled with an siRNA can be used. Specific examples of fluorescent labels include Cy3™, Cy5™ (Amersham), other cyanine derivatives, FITC, one of the ALEXA™ or BODIPY™ dyes (Molecular Probes, Eugene, OR), a dabsyl moiety, and the like. It is also possible to use fluorescent microparticles, such as inorganic fluorescent particles as long as the particle has a size that does not affect transfection efficiencies. The labels may be used to visualize the distribution of the labeled siRNA within a transfected cell. In addition, the label can be used to distinguish between transfected cells from non-transfected cells. As such, a population of cells can be transfected with the labeled siRNA and sorted by FACS. Moreover, the fluorescent labels can be particularly well suited for HCS and HTC analytical techniques. For example, cells that have been transfected can be identified, and then be further examined using HCS analysis.

In one embodiment, the label can be a luminescent moiety other than a fluorescent label. Such luminescent moieties can include phosphorescent microparticles that can be coupled to siRNA. Preferably, the size of the phosphorescent microparticle does not affect transfection. Alternatively, the luminescent label can be a chemiluminescent moiety. A chemiluminescent label can produce light by a chemical or electrochemical reaction. Chemiluminescence usually involves the oxidation of an organic compound, such as luminol or acridinium esters, by an oxidant like hydrogen peroxide or hypochlorite. Also, chemiluminescent reactions can occur in the presence of catalysts such as alkaline phosphatase, horseradish peroxidase, metal ions or metal complexes. As such, a control siRNA can be labeled with a chemiluminescent organic compound for transfection, wherein the transfection efficacy is measured by chemiluminescence after the catalyst is added to the contents of the transfected cell.

The use of labeled nucleotides is well known to persons of ordinary skill, and labels such as enzymatic, mass, or radioactive labels, may be used in applications in which such types of labels would be advantageous. Further descriptions of labels that are applicable for transfection control siRNA in RTF testing protocols are found in U.S. Provisional Patent Application No. 60/542,646, 60/543,640, and 60/572,270 and International Application PCT/US04/10343 (Published as WO 2004/090105).

The label can be attached directly to the control siRNA or through a linker. The label can be attached to any sense or antisense nucleotide within the siRNA, but it can be preferable for the coupling to be through the 3' terminal nucleotide and/or 5' terminal nucleotide. An internal label may be attached directly or indirectly through a linker to a nucleotide at a 2' position of the ribose group, or to another suitable position. For example, the label can be coupled to a 5-aminoallyl uridine.

For example, linkers can comprise modified or unmodified nucleotides, nucleosides, polymers, sugars, carbohydrates, polyalkylenes such as polyethylene glycols and polypropylene glycols, polyalcohols, polypropylenes, mixtures of ethylene and propylene glycols, polyalkylamines, polyamines such as polylysine and spermidine, polyesters such as poly(ethyl acrylate), polyphosphodiesters, aliphatics, and alkylenes. An example of a conjugate and its linker is cholesterol-TEG-phosphoramidite, wherein the cholesterol is the conjugate and the tetraethylene glycol ("TEG") and phosphate serve as linkers.

In one embodiment, the transfection control siRNA can be a type where its presence is identified or measured by the effects on a cell. The control siRNA can be detected by an established and reproducible effect that is caused by the presence of the control siRNA in a cell. For example, the control siRNA can be a type that causes cell toxicity and/or death when transfected into a cell, or can cause an identifiable or measurable response not related to RNAi. Cytotoxic siRNA can be used as transfection control siRNA because the amount of cell toxicity can be directly related to the siRNA. The gene silencing efficacy of a test well can be identified by the amount of toxicity induced by the cytotoxic siRNA in the control well. Also, the effect of the cytotoxic siRNA can be compared to cells in blank wells as well as to cells in test wells. In the instance the cytotoxic siRNA induces cell death and the cells in the blank wells or test wells do not show any toxic effects, the transfection and/or gene silencing efficacy of the test well can be compared to level of cell death induced by the cytotoxic siRNA. However, established and reproducible toxic effects that arise from cytotoxic siRNA can be analyzed without being compared to cells in other wells.

In one embodiment, the transfection control siRNA is a cytotoxic siRNA. Some cytotoxic siRNA which have been identified include sense polynucleotide sequences that include the following motifs: AAA; UUU; GCCA; or UGGC. Also, an siRNA can be toxic by including a long polynucleotide sequence. The toxic control siRNA can include a long dsRNA that is about 50 base pairs, and preferably longer than 50 base pairs. Optionally, the toxic control siRNA can induce an interferon response that is toxic to the cells. Cytotoxic transfection control siRNA can be represented by Dharmacon's siCONTROL™ TOX. Additionally, siRNA that induce a toxic response through the RNAi pathway by inhibiting production of a protein vital to cell viability can be used as cytotoxic siRNA.

**B. Positive Control siRNA**

Positive control siRNA can be well characterized siRNA that silence a well known gene. That is, the control siRNA silence a known gene with established and reproducible results. As such, the positive control siRNA can provide meaningful data regarding the efficacy of transfection and/or gene silencing in a test plate having a similar RTF protocol or conditions. In part, this is because the control siRNA are known to systematically silence a known gene to stop production of a known protein, wherein the known gene and known protein can be referred to as a control gene and control protein or control polypeptide, respectively.

Positive control siRNA can be distinguished from test siRNA by a number of characteristics. Usually, test siRNA are being tested to identify whether or not a target test gene will be silenced in an RTF protocol or condition. On the other hand, positive control siRNA have well known sequences and/or characteristics that systematically silence a well known gene in a reproducible and measurable manner. This allows the positive control siRNA to provide meaningful results regarding the efficacy of the test siRNA because when the positive control gene is not silenced or is overly silenced in the control well, the results obtained from a test well from using the test siRNA may be unreliable. Examples of positive control siRNA include siRNA that can silence MAP kinase genes, glyceraldehyde-3-phosphate dehydrogenase ("GAPDH") gene, cyclophilin B ("cyclo") gene, Lamin A/C genes, and other well established genes that can be silenced with siRNA having specific polynucleotide sequences. Specific examples of positive control siRNA include Dharmacon's siCONTROL™ GAPD, siCONTROL™ Cyclophilin B, and siCONTROL™ Lamin A/C.

Positive control siRNA can also include siRNA that inhibit the RNAi pathway, which uses siRNA targeting RISC genes, Ago2 genes, eIF2C2 genes, and the like for silencing. Additionally, these positive control siRNA can silence human, mouse, rat, or other animal control genes. In addition, positive control siRNA can target any gene that when silenced, gives a predictable result in e.g. a phenotypic screen.

In one embodiment, the positive control siRNA can be selected to optimize functionality in silencing the control gene. Preferably, the positive control siRNA has between 50% and 100% gene silencing functionality with respect to the control gene, more preferably between 70% and 100%, even more preferably between 80% and 100%, and most preferably between 90% and 100% functionality in silencing the control gene.

Additionally, the positive control siRNA antisense strand can have varying levels of complementarity with the control sequence to which it targets (*e.g.*, control mRNA or gene). Preferably, the antisense strand can have about 50-100% complementarity with the target sequence, more preferably, about 70-100% complementarity, even more preferably about 80-100% complementarity, still even more preferably about 90-100% complementarity, and most preferably about 100% complementarity with the control sequence.

In one aspect, the positive control siRNA can be selected by rational design. As such, the positive control siRNA is selected using one or more formulas that identify sequences that have desirable attributes and are more highly functional. Highly functional positive control siRNA can be identified by rational design in order to perform more consistently and reproducibly than less functional siRNA under a wide range of conditions found in RTF formats (*e.g.*, cell densities).

In one embodiment, it can be preferable to select positive control siRNA that have been previously identified from lists of siRNA that have been selected using rational design algorithms. As such, the control siRNA can be selected from Table I of the provisional application having Serial No. 60/678,165. Table I is entitled "siGENOME Sequences for Human siRNA," and consists of columns "Gene Name," "Accession No.," "Sequence," and "SEQ. ID NO." Table I lists 92,448 19-mer siRNA sense strand sequences, where antisense strand sequences were omitted for clarity. The siRNA sequences listed in Table I includes SEQ. ID NOs. 1-92,448, wherein each preferably can also include a 3' UU overhang on the sense strand and/or on the antisense strand. Each of the 92,448 sequences of Table I, when used in an siRNA, can also comprise a 5' phosphate on the antisense strand. Of the 92,448 sequences listed in Table I, 19,559 have an on-targeting set of modifications. A list of sequences, identified by SEQ. ID NO., that have on-target modifications is presented in Table II, entitled "List of Table I Sequences Having On-Target Modifications Identified by SEQ. ID NO." On-target modifications are on SEQ. ID NOs. 1-22,300.

## C. Negative Control siRNA

Negative control siRNA can be characterized as not being functional within the RNAi pathway, and thereby do not induce gene silencing. This can be accomplished by various mechanisms which include siRNA that are not functional, siRNA that inhibit uptake and processing by RISC, and/or siRNA that do not have complementarity to a gene or mRNA. For example, the negative control siRNA can fail to enter RISC. As such, negative control siRNA can provide meaningful results that are well known and reproducible by not affecting the cellular processes of transfected cells. Also, negative control siRNA can provide indications of gene silencing conditions and protocols by testing for any induced toxicity, loss of cellular function, or non-specific inhibition of protein production.

In one embodiment, the negative control siRNA is non-functional siRNA, which does not function in the RNAi pathway. The non-functional siRNA can provide an indication of transfection efficacy by monitoring the response of the transfected cell. Instances in which non-functional siRNA cause changes in gene expression, cell function, or phenotype may be the result of factors other than RNAi mediated gene silencing, and can provide an indication regarding the efficacy of gene silencing of test wells having substantially similar RTF protocols and/or conditions. Non-functional siRNA can have certain sequences and/or chemical modifications in order to induce non-functionality. Additionally, non-functional siRNA can have a 17 base pair duplex or any duplex having less than 18 base pairs. For example, non-functional siRNA can include a 17 base pair duplex with 2' modifications at the first and second sense nucleotides and on the first and second antisense nucleotides. In another example, a non-functional siRNA can include a 19 base pair duplex with 5' deoxynucleotides on the 5' end of the sense and the antisense strands.

In one embodiment, the negative control siRNA includes sequence and/or modifications that inhibit uptake and processing by RISC. A negative control siRNA can include a modification or size that inhibits being taken in and processed by RISC. That is, RISC is not able to perform a function with mRNA having the sequence of the negative control siRNA, wherein the negative control has a modification or size that prevents being taken into RISC. This can be used to modify siRNA having selected sequences that can be used for comparative purposes with test siRNA with the same sequence but not having the modifications. As such, any gene silencing that arises from the negative control siRNA may result from non-specific silencing and provide an indication that the data obtained from the test siRNA is not reliable. An example of the negative control siRNA that are not taken in or processed by RISC can include siCONTROL™ RISC-Free (Dharmacon, Inc.).

For example, the negative control siRNA that are non-functional or are not taken in and processed by RISC can have various 2' modifications. This can include control siRNA that contain 2' modifications on the first and second sense nucleotides, 2' modifications on at least one through all pyrimidine sense nucleotides, 2' modifications on the first and/or second antisense nucleotides, 2' modifications on at least one through all pyrimidine antisense nucleotides, and/or a 5' carbon having a phosphate modification at the sense or antisense 5' terminal nucleotide. The 2' modifications can be 2'-O-aliphatic modifications or 2'-halogen modifications. The control siRNA can also include internucleotide modifications with phosphorothioates or methylphosphonates.

The 2' modifications can be characterized by as 2'-O-aliphatic modifications such as 2'-O- alkyl modifications. For example, the 2'-O-alkyl can be selected from the group consisting of 2'-O-methyl, 2'-O-methyl, 2'-O-propyl, 2'-O-isopropyl, 2'-O-butyl, 2'-O-isobutyl, 2'-O-ethyl-O-methyl (*i.e.,* $-CH_2CH_2OCH_3$), 2'-O-ethyl-OH (i.e., $-OCH_2CH_2OH$), 2'-orthoester, 2'-ACE group orthoester, and combinations thereof. Most preferably, the 2'-O-alkyl modification is a 2'-O-methyl moiety. Additionally, the 2'-halogen modifications can be selected from the group consisting fluorine, chlorine, bromine, or iodine; however, fluorine is preferred.

In one embodiment, the negative control siRNA can be an siRNA that has a sense and/or antisense strand with limited or non-functional complementarity to a gene or mRNA sequence. This can include siRNA that are bioinformatically designed to minimize any potential targeting of any known human or animal gene. The non-targeting negative control siRNA can modified or unmodified, and examples include Dharmacon's siCONTROL™ Non-Targeting siRNA #1 and siCONTROL™ Non-Targeting pool.

**D. Dual Function Control siRNA**

In one embodiment, the present invention includes dual function control siRNA. A dual function control siRNA can be used for two different control studies. This can include a single control siRNA that functions both as a transfection control and a positive control. Also, this can include a single control siRNA that functions both as a transfection control and a negative control. Examples of dual function controls include any positive or negative control that includes a label, such as a fluorescent label (*e.g.*, siGLO™ Cyclophilin B, siGLO™ Lamin A/C, siGLO™ RISC-Free, each from Dharmacon, Inc.).

**E. Control siRNA Configurations**

The control siRNA may be used individually (*e.g.*, one siRNA sequence per well) or as part of a pool. A pool of control siRNAs, as defined herein, refers to the use of at least two different control siRNAs within a specific well, and usually at least four different control siRNAs. Each control siRNA can include between 18 and 31 base pairs, more preferably between 19 and 26 base pairs, and most preferably 19 and 21 base pairs. However, toxic control siRNA can have duplex regions with about 50 base pairs or greater than 50 base pairs, and some non-functional control siRNA can include less than 18 base pairs. Each control siRNA can include a sense strand and an antisense strand, which are preferably at least substantially complementary to each other over the range of the duplex region. It is most preferable for the duplex region to have about 100% complementarity.

Additionally, the control siRNA can have overhangs, bulges, mismatches, stability modifications, specificity modifications, hairpin structures, or other common features on target siRNA. Accordingly, additional information regarding these features can be reviewed in the cross-referenced patent application filed herewith having Attorney Docket No. 16542.1.1, entitled APPARATUS AND SYSTEM HAVING DRY GENE SILENCING COMPOSITIONS, with Barbara Robertson, Ph.D., et al. as inventors.

A reduction in off-targeting or increased specificity can also be achieved by using control siRNA concentrations that are below the level that induces off-target effects. As an example, transfection of a single control siRNA at 100 nM can induce 90% silencing, yet the high concentration of the siRNA may also induce off-target effects. In contrast, a pool of four control siRNAs (*e.g.*, total concentration of 100 nM, 25 nM each) can similarly induce 90% silencing. Since each siRNA is at a fourfold lower concentration, the total number of off-targets is fewer. Thus, in order to obtain control gene silencing with inhibited or no off-target effects, a highly functional siRNA can be used at low concentrations, or pools of control siRNA targeting the same gene can be used with each siRNA of the control pool having a concentration that is sufficiently low to minimize off-target effects. Preferably, the total amount of control siRNAs can be delivered at concentrations that are less than or equal to about 100 nM, more preferably less than or equal to about 50 nM, even more preferably less than or equal to about 25 nM, and most preferably less than or equal to about 10 nM.

### VII. Polynucleotide Carriers

In one embodiment, the present invention includes polynucleotide carriers that can interact with a control siRNA, and transport the control siRNA across a cell membrane. However, in other embodiments of the invention modes of transfection can be implemented without carriers, such as by electrophoresis, precipitation, particle bombardment, optoporation, and microinjection. Usually, polynucleotide carriers include a positive charge that interacts with the negatively charged phosphates on the polynucleotide backbone. Polynucleotide carriers are well known in the art of cellular nucleic acid deliver. Preferred polynucleotide carriers include polymers, lipids, lipopolymers, lipid-peptide mixtures, and the like that are capable of complexing with an siRNA and delivering the siRNA into a cell in a manner that retains the gene silencing functionality without being overly toxic. As such, routine experimentation can be implemented with procedures described herein with respect to optimizing RTF protocols in order to identify the optimal polynucleotide carrier for a certain system or cell.

In one embodiment, lipids or lipid-peptide mixtures are preferable for introducing siRNA into a target cell. Typically, the lipid is a cationic lipid. Cationic lipids that can be used to introduce siRNA into cells can be characterized by having little or no toxicity (*e.g.*, defined as less than 15-20% toxicity), which can be measured by AlamarBlue or equivalent cell viability assays. However, not all lipids are functionally equivalent and certain lipids can perform better with specific cell lines. Thus, the foregoing optimization procedures can be employed to determine an appropriate lipid and lipid concentration for delivering siRNA for a specific cell line. Peptides that have affinity to one or more proteins, lipids, lipid-polysaccharide, or other components of the cell membrane can be conjugated to the siRNA and used independent of lipids or advantageously combined with one or more lipids to form a polynucleotide carrier. Such lipid-peptide mixtures can enhance RTF of siRNA. Cholesterol conjugates can be similarly coupled to the siRNA and be used independent of polynucleotide carriers or advantageously combined therewith.

Briefly, in order to identify whether a given lipid is acceptable for siRNA RTF testing protocols, two or more well characterized control siRNAs can be tested under a variety of lipid, media, and siRNA concentrations using the optimizing protocols described herein. Subsequently, the level of transfection and/or gene silencing and/or the level of cell death are quantified using art-accepted techniques. Suitable lipids for siRNA RTF testing protocols include OLIGOFECTAMINE™, TransIT-TKO™, or TBIO Lipid 6™, LIPOFECTAMINE™ 2000, lipids Dharma*FECT*™ 1, Dharma*FECT*™ 2, Dharma*FECT*™ 3, and Dharma*FECT*™ 4 (Dharmacon, Inc.). The term "Dharma*FECT*™ (followed by any of the numerals 1, 2, 3, or 4) or the phrase "Dharma*FECT*™ transfection reagent," refers to one or more lipid-based transfection reagents that have been optimized to transfect siRNA rather than larger nucleic acids (*e.g.*, plasmids). Additional information on lipids can be obtained in U.S. Patent Nos. 5,674,108, 5,834,439, 6, 110,916, 6,399,663, and 6,716,582, and international publications WO 00/12454 and WO 97/42819.

The formation of a functional control siRNA-lipid complex can be prepared by combining control siRNA and the lipid. As such, an appropriate volume of lipid at a selected concentration can be combined with a volume of media and/or buffer to form a lipid-media or lipid-buffer having a suitable concentration of lipid. For example, a volume of lipid media ranging from about 5-50 microliter ("uL") can include about 0.03-2 micrograms ("ug") of lipid to be introduced into each well of a 96-well plate, and the amount of lipid can be changed to correspond with other well sizes. The choice of media and/or buffer can improve the efficiency of the RTF protocol. Some media contain one or more additives that induce cell toxicity and/or non-specific gene modulation during RTF testing protocols. Examples of preferred media or buffers include Opti-MEM™ (GIBCO, Cat. # 31985-070), HyQ-MEM-RS™ (HyClone, Cat.# SH30564.01), Hanks Balanced Salt Solution™, or equivalent media. A suitable media can be identified by employing the optimization protocol described herein.

The lipid-media or lipid-buffer can be introduced into a well by a variety of methods including hand-held single and multi-channel pipettes, or more advanced and automated delivery systems that can inject measured volumes of the lipid solution into a well. The lipid solution can be incubated in the well that contains the dried control composition for a period of time that is sufficient to solubilize or suspend the siRNA, and to form control complexes. In general, the process of siRNA control solubilization and complex formation can require about 20 minutes, but usually not more than 120 minutes. The complex formation process is generally performed at room temperature, but can be performed at temperatures ranging from 4-37 °C. In some instances, the lipid and control siRNA can be mixed by agitating the plate (*e.g.*, swirl, vortex, sonicate) for brief periods (*e.g.*, seconds - minutes) to enhance the rate of control siRNA solubilization and complex formation.

Additionally, any of the foregoing polynucleotide carriers can be included in systems or kits in accordance with the present invention. Such kits can include the plates having control compositions, and can be distributed with siRNA solubilizing or suspending solutions, polynucleotide carriers, carrier solutions, reagents, cell media, and the like.

## VIII. Well Arrangements

In one embodiment, the siRNA RTF testing plates that include multiple wells having different dry control compositions can have the wells organized into predefined arrangements. Such arrangements can correspond to the type of assay being employed with the siRNA RTF testing plate. That is, when a family of genes is being studied, a first control siRNA can be organized in one column or row while a second control or targeting siRNA can be organized in a different column or row. Thus, the wells can be organized into a pre-selected arrangement so that particular control siRNAs are in a pre-selected pattern on a plate. The pre-selected pattern can include various control wells, such as those that include one or more negative, positive, and/or transfection controls. Also, the pre-selected pattern can include wells that are empty or substantially devoid of any siRNA, which can be used as controls and for calibrations.

It can be beneficial to have control siRNAs that are pre-dried in corresponding wells of different well plates so that multiple RTF testing plates can be prepared simultaneously. This can allow for RTF testing plates to have gene silencing compositions at standardized positions and amounts of control siRNAs, which is beneficial for using standardized well plates in multiple experiments that can be conducted over time without introducing variability between the plates. The use of standardized plate arrangements can provide a series of plates that can be used over time and provide data that can be analyzed together.

For example, a plate comprising a plurality of columns of wells can include a transfection control in the first column, positive controls for RNAi in the second column, negative controls for RNAi in a third column, a pool of siRNAs directed against a single target in a fourth column, and individual members of the siRNA pool that comprise the fourth column are in subsequent columns, such as the fifth through twelfth columns. Alternatively, the fifth through twelfth columns can comprise different concentrations of each siRNA in the pool of the fourth column, with the amount of siRNA increasing from well to well or decreasing from well to well. Each well can include one concentration of each siRNA in the pool, or two, three, four, five, or more concentrations of each siRNA in the pool can be in different wells. The number of concentrations of siRNA that can be used is limited only by the number of wells on the plate; however, multiple plates can be configured to be used together with a predefined pattern that spreads across all the plates. Additionally, the pre-selected patterns of control siRNA concentration gradients can be used as a pattern that can be observed so that the optimal amount of each control siRNA can be determined by observing the level of transfection and/or gene silencing number of concentrations of that particular control siRNA.

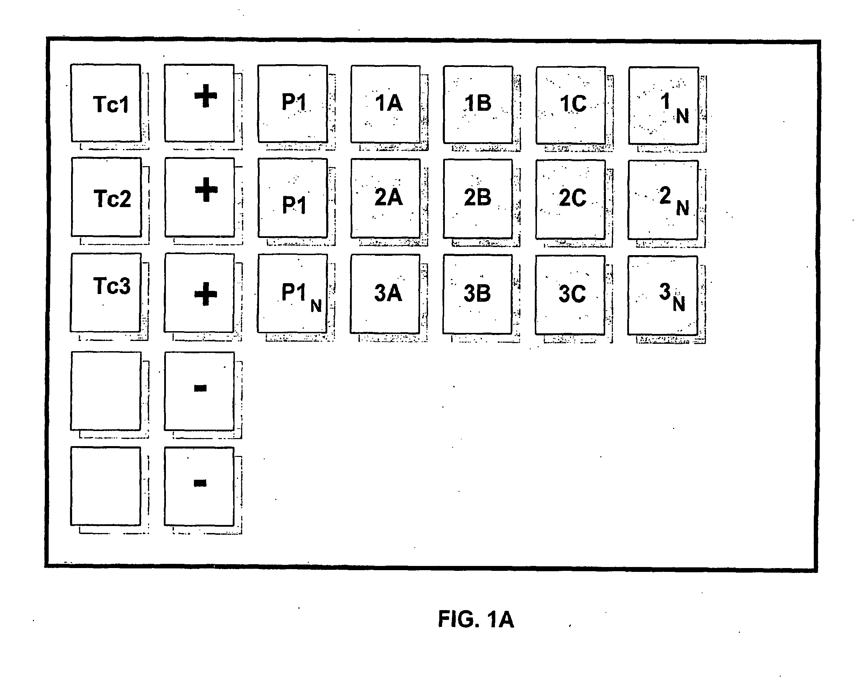

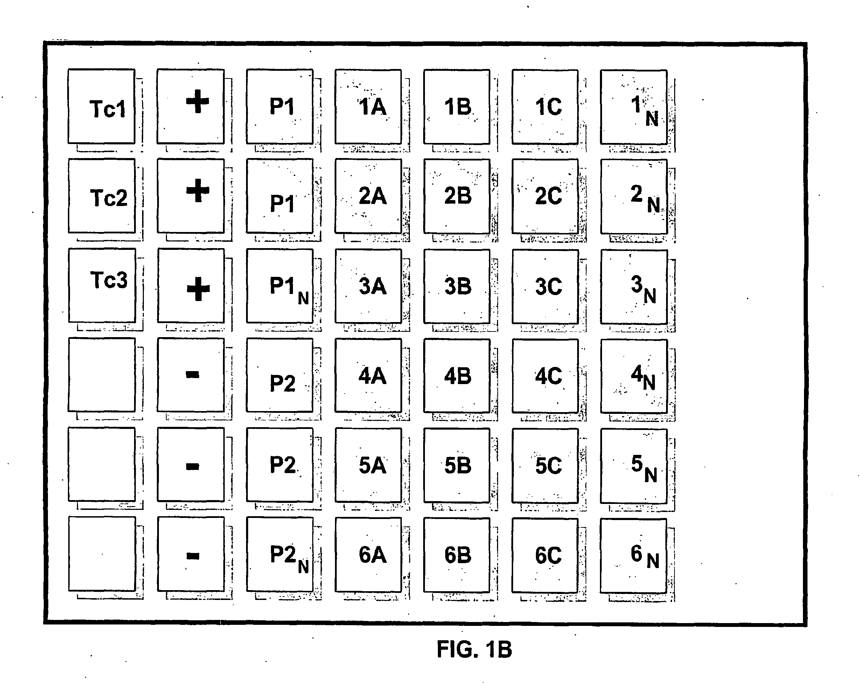

Figures 1A and 1B illustrate embodiments of plate arrangements similar with the foregoing concentrations arrangements. While the wells are shown to be square, it should be recognized that they can be any shape. Also, the well plate can include any number of wells, and the number of wells depicted is merely for example. In the figures the wells are defined as follows: "Tc" indicates a transfection control well, wherein the increasing corresponding numbers identify different transfection controls; blank wells indicate wells devoid or substantially devoid of any siRNA; "+" indicates a positive control; "-" indicates negative controls; "P1" through "$P1_N$" indicate a first pool which silences a first gene at a concentration gradient; "P2" through "$P2_N$" indicate a second pool which silences a second gene at a concentration gradient; "1A" through "$1_N$" indicate a first individual test siRNA of the first pool at a concentration gradient; "2A" through "$2_N$" indicate a second individual test siRNA of the first pool at a concentration gradient; "3A" through "$3_N$" indicate a third individual test siRNA of the first pool at a concentration gradient; "4A" through "$4_N$" indicate a first individual test siRNA of the second pool at a concentration gradient; "5A" through "$5_N$" indicate a second individual test siRNA of the second pool at a concentration gradient; and "6A" through "$6_N$" indicate a third individual test siRNA of the second pool at a concentration gradient Thus, Figure 1A illustrates a well plate assaying a single pool, and Figure 1B illustrates a well plate assaying multiple pools. Additionally, a well plate can include more than two pools.

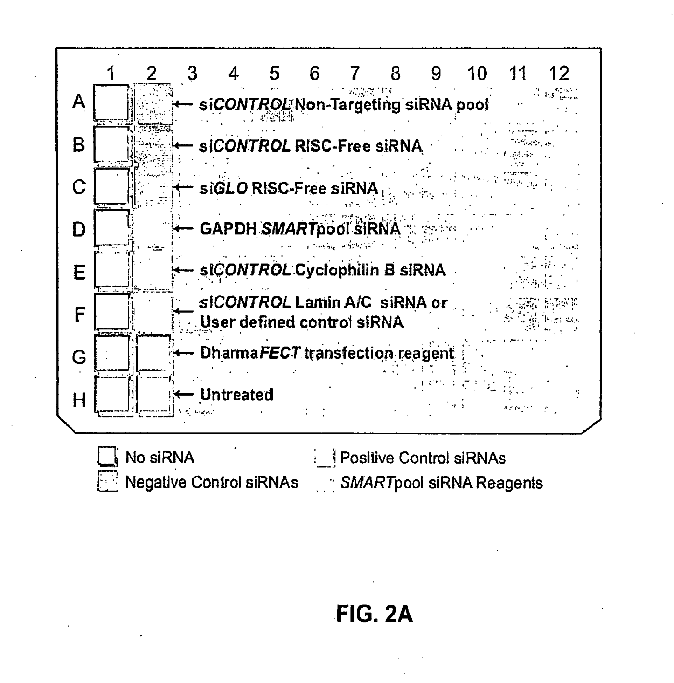

Figure 2A is a schematic diagram that illustrates an embodiment of a well plate having control siRNA. More particularly, wells A1-H1, G2, and H2 are blank wells devoid of siRNA. For example, wells A1-H1 are available for RTF testing controls. Well G2 can be used as a mock transfection control, and well H2 can be an untreated cell control. Wells A2-F2 contain negative and positive transfection control siRNAs. More particularly, the control wells include the following: well A2 includes a non-targeting negative control siRNA (*e.g.*, siCONTROL™ Non-Targeting siRNA pool from Dharmacon, Inc.); well B2 includes an siRNA that inhibits being taken in and processed by RISC (*e.g.*, siCONTROL™ RISC-Free from Dharmacon, Inc.); well C2 includes a dual control siRNA that can be used as a negative control and a transfection control (*e.g.*, siGLO™ RISC-Free from Dharmacon, Inc.); well D2 includes a positive control siRNA targeting GAPDH control gene (*e.g.*, siCONTROL™ GAPD from Dharmacon, Inc.); well E2 includes a positive control siRNA targeting cyclophilin B control gene (*e.g.*, siCONTROL™ Cyclophilin B siRNA from Dharmacon, Inc.); and well F2 is a positive control siRNA targeting Lamin A/C control gene (*e.g.*, siCONTROL™ Lamin A/C from Dharmacon). The wells in columns 3-12 can each contain a test siRNA or test siRNA pool such as any of Dharmacon's *SMART*pool™ siRNA reagents. Alternatively, well F2 can be a user defined control siRNA.

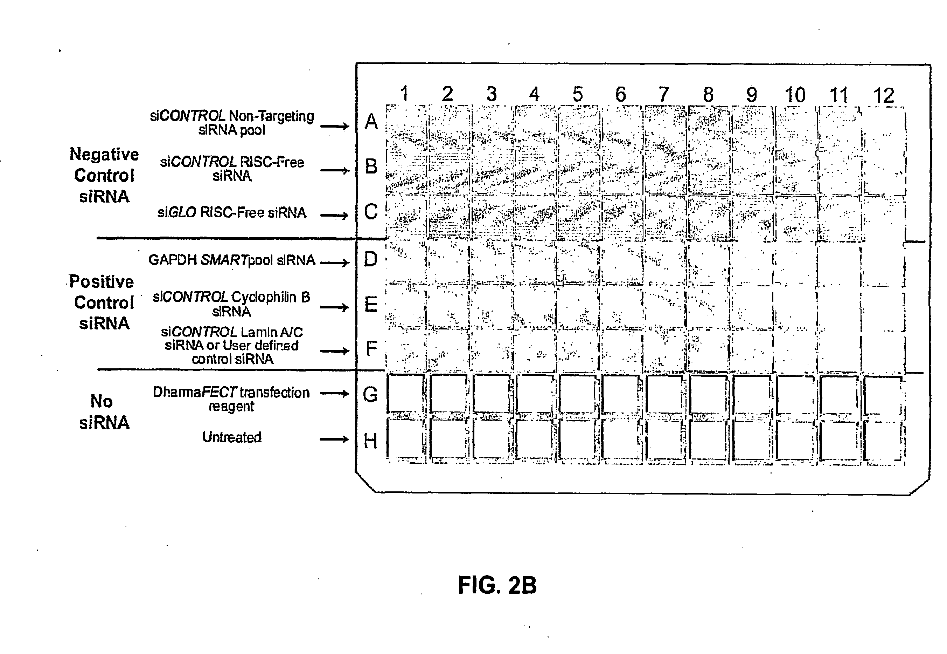

Figure 2B is a schematic diagram that illustrates an embodiment of an optimization well plate or an RTF testing plate having control siRNA. As shown, rows A-C can contain three negative control siRNAs (*e.g.*, siCONTROL™ Non-Targeting siRNA pool, siCONTROL™ RISC-Free siRNA, siGLO™ RISC-Free siRNA, each from Dharmacon, Inc.). Rows D-E can contain three positive transfection control siRNAs (*e.g.*, siCONTROL™ GAPD, siCONTROL™ Cyclophilin B siRNA, siCONTROL™ Lamin A/C siRNA, each from Dharmacon, Inc.). Alternatively, Row F can be a user defined control siRNA. Row G does not contain siRNA and can be used for mock-transfected cells in order to study the effect of the transfection reagent alone on cell viability and/or mRNA expression. Row H does not contain siRNA and is can be used for untreated cells, and can serve as a 100% viability control and/or 100% mRNA level control.

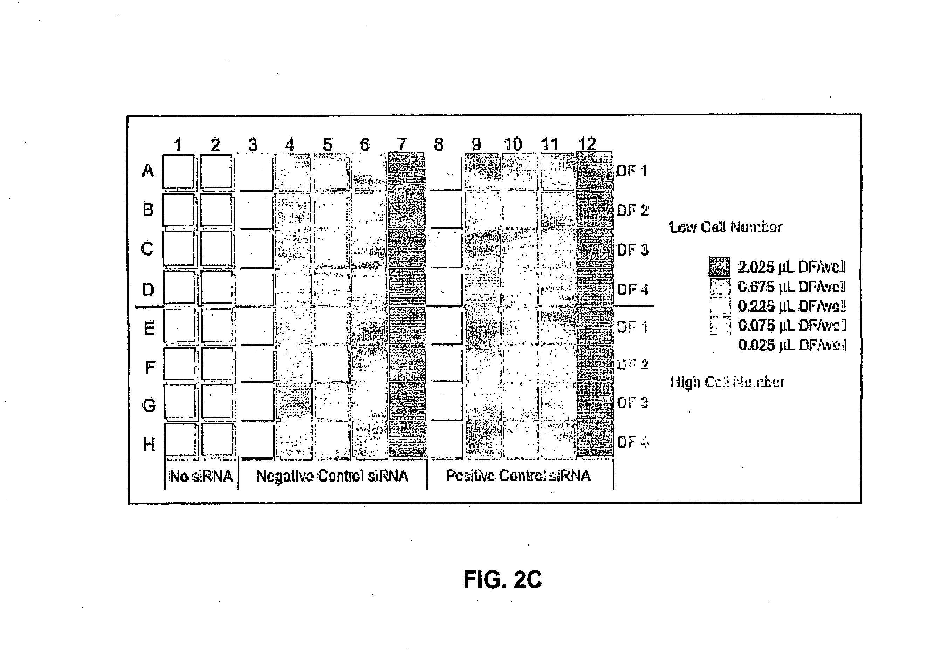

Figure 2C is a schematic diagram that illustrates an embodiment of an optimization plate or an RTF testing plate having control siRNA. The testing plate includes an arrangement of wells that can be used in a procedure for optimizing RTF conditions. As shown, column 1 includes blank wells devoid of siRNA, and can be used as a blank that does not receive cells. This column can be used for a standard curve or other experimental controls that may be desired for an RTF testing protocol for assessing the efficacy of gene silencing. Column 2 includes blank wells devoid of siRNA, and can be used as a blank that does receive cells. This column can be used as an untreated reference for different volumes of polynucleotide carrier, such as Dharma*FECT*™ transfection reagent, which is tested in columns 3-12. Columns 3 - 7 can contain negative control siRNAs, which serve as negative control samples for each Dharma*FECT*™ transfection reagent volume used. As shown, up to five volumes of each Dharma*FECT*™ transfection reagent may be tested in one plate. In each row, the Dharma*FECT*™ transfection reagent volumes in columns 3-7 can be repeated in columns 8-12. This can allow the negative control siRNAs to serve as references for any gene silencing that occurs in the wells of columns 8-12, which contain positive transfection control siRNAs. Additionally, rows A-D can be seeded with a low cell density, and rows E-H can be seeded with a high cell density. Thus, the plate can include a combination of different RTF conditions, which can be used to determine the optimal amount of Dharma*FECT*™ transfection reagent, Dharma*FECT*™ transfection reagent volume, and cell number.

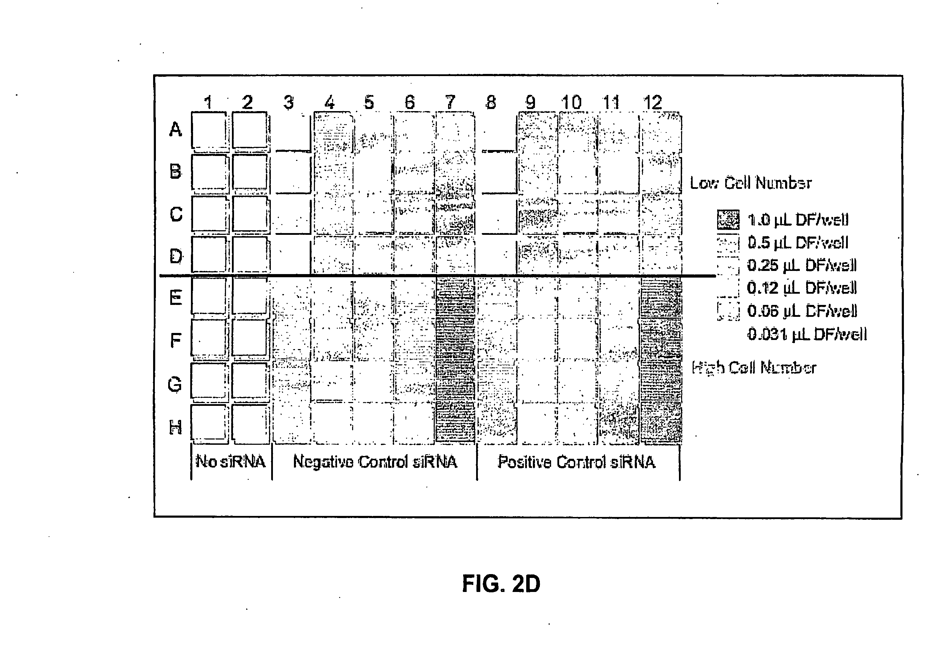

Figure 2D is a schematic diagram that illustrates an embodiment of an optimization well plate or an RTF testing plate having control siRNA. As shown, the plate is arranged with blanks, negative control siRNA, and positive control siRNA as in Figure 2C. However, this plate can be assayed with different polynucleotide carrier concentrations. Rows A-D can be used with low cell densities with Dharma*FECT*™ transfection reagent at volumes ranging from 0.03 uL/well (*e.g.*, columns 3 and 8) to 0.5 uL/well (*e.g.*, columns 7 and 12). Rows E-H can be used with high cell densities with Dharma*FECT*™ transfection reagent at volumes ranging from 0.06 uL/well (*e.g.*, columns 3 and 8) to 1.0 uL/well (*e.g.*, columns 7 and 12).

## EXAMPLES

The following examples are provided to describe some embodiments of the present invention in a manner that can be use by one of skill in the art to practice the present invention. Additionally, the following examples include experiments that were actually performed as well as prophetic experiments. Additional examples and supplementary information for the following examples can be reviewed in the incorporated references having Attorney Docket No. 16542.1.1 (Publication No: US-2006-0115461), entitled APPARATUS AND SYSTEM HAVING DRY GENE SILENCING COMPOSITIONS, with Barbara Robertson, Ph.D., et al. as inventors, Attorney Docket No. 16542.1.2 (Publication No: US-2006-0110829), entitled APPARATUS AND SYSTEM HAVING DRY GENE SILENCING POOLS, with Barbara Robertson, Ph.D., et al. as inventors, and U.S. Provisional Application Serial No. 60/678,165. The polynucleotide sequences that were used in the examples can be found in Tables I-IV of U.S. Provisional Application Serial No. 60/678,165, and the sequence listing of the reference having Attorney Docket No. 16542.1.1 (Publication No: US-2006-0115461), entitled APPARATUS AND SYSTEM HAVING DRY GENE SILENCING COMPOSITIONS, with Barbara Robertson, Ph.D., et al. as inventors.

## Example 1

The effect of plate conditions on RTF protocols was assayed in order to determine optimum conditions. The different types of plate coatings that were studied included untreated, fibronectin-treated, poly-L-Lysine treated, MATRIGEL™-treated, and CELLBIND™ plates. In the poly-L-lysine plates, each well was treated with 50 uL of 5 ug/uL poly-L-lysine for 1 hour, and washed with ddH$_2$O (3x) and dried under a UV light for 20 minutes. MATRIGEL™ plates were obtained from BD Biosciences (Catalog No. 354607, Bedford, MA). Fibronectin plates were purchased from Becton Dickinson Labware (Biocoat cellware, Catalog No. 354409, Bedford, MA) and CELLBIND™ plates were obtained from Coming (Catalog No. 3300). For each study, varying amounts of human cyclophilin B siRNA (*e.g.*, cyclo3) were added to different wells at 0-250 nM.

The results of these studies are provided in Figures 3A-3J, which are graphs illustrating the efficacy of the RTF testing procedures. The graph of Figure 3A depicts the cell viability of the untreated plates, wherein the cell viability drops fairly steadily as the lipid concentration increases. At low lipid concentrations (*e.g.* 0.125 ug lipid per 100 uL) the cells were sufficiently viable to provide meaningful gene silencing results. The graph of Figure 3B depicts the cyclophilin B siRNA successfully silenced the target gene. The graph of Figure 3C depicts the cell viability of polylysine plates to similarly decrease as the lipid concentration increase, and the lower lipid concentrations were not overly toxic to the cells. The graph of Figure 3D depicts the cyclophilin B siRNA successfully silenced the target gene. The graph in Figure 3E depicts the CELLBIND™ plates to have reduced toxicity with acceptable levels of cell viability being preserved up to 0.25 ug of lipid per 100 uL. However, the graph of Figure 3F shows that gene silencing was only moderate, and was determined to be unacceptable. The graph in Figure 3G depicts the MATRIGEL™ plates to have overall poor cell viability under all conditions, and the graph of Figure 3H was considered to be unreliable. The graph of Figure 3I depicts the fibronectin-coated plates to also have poor cell viability, and the graph of Figure 3J was considered to be unreliable.

## Example 2

The optimization of RTF protocols to induce gene silencing was studied by assessing the siRNA functionality in relation to cell density. The siRNAs of varying functionalities (*e.g.*, F50- F95) were reverse transfected under a range of cell densities (*e.g.*, 10,000-40,000 cells per well) using lipid concentrations that induced minimal levels of cell toxicity (0.063 ug of lipid per 100 uL for 10,000 cells per well, 0.125 ug of lipid per 100 uL for 20,000 cells per well, and 0.25 ug of lipid per 100 uL for 40,000 cells per well). The study was performed with control cyclophilin B siRNA (*e.g.*, cyclo3, cyclo28, and cyclo37 with functionalities of 95, 75, and 50, respectively).
Figure 4 is a graphical representation of the gene silencing of increasing concentrations of siRNA in relation to the increasing number of cells. The graph shows that highly functional siRNA induced greater gene silencing under a broad range of conditions. The cyclo3 siRNA induced 60% or more silencing at all three cell concentrations. In contrast, less functional molecules (*e.g.*, cyclo 28) performed well at only low cell densities (*e.g.*, 10, 000 cells per well), but less well at higher cell densities (*e.g.*, 20-40,000 cells per well). Thus, increased functional siRNA, which are rationally designed greatly improve gene silencing.

## Example 3

A population of randomly selected siRNAs derived from a walk targeting DBI (*e.g.*, NM_020548, position 202-291) was assessed for the ability to induce toxicity. The collection of siRNAs consisted of 90 individual (*e.g.*, 19 bp) duplexes, and covered the respective regions in single base steps. Duplexes were forward transfected into HeLa cells using LIPOFECTAMINE™ 2000, and a threshold of 75% cell viability was used as the cutoff to distinguish toxic from nontoxic sequences.
Figure 5A is a graphical representation of the results of the toxicity study. As shown, the siRNAs transfected under these conditions were observed to induce varying levels of cellular toxicity. Overall, 14 out of 90 siRNA duplexes (*e.g.*, 15.5%) were found to decrease cellular viability below 75%, which is identified by the horizontal dashed line. These toxic siRNA can be identified by the numbers within the boxes that show cell survival below the dashed line, and can be used as transfection control siRNA
Figure 5B is a graphical representation of an the cell toxicity and viability obtained from individual siRNAs of 48 functional (*e.g.*, >70% silencing) pools of four siRNA targeting 12 different genes. Only twelve of the forty-eight sequences (*e.g.*, 25%) decreased cellular viability below 75%.
Figure 5C is a graphical representation of the toxicity of exemplary siRNA of the pools depicted in Figure 5B. While all eight duplexes targeting MAP2K1 and MAP2K2 show greater than 80% gene silencing, only a single siRNA in each quartet reduces cell viability below 75% (*e.g.*, MAP2K1-d4 and MAP2K2-d3). Thus, as the remaining siRNAs in each group were equally functional, but non-toxic, the toxicity induced by MAP2K1-d4 and MAP2K2-d3 is unrelated to target knockdown.
A linear display of the distribution of toxic siRNA along the DBI walk showed that the dispersal of these toxic sequences was frequently non-random (i.e., clustered) and suggested the presence of one or more motifs that were responsible for the observed toxicity (*e.g.*, see boxed areas of Figure 5A). Subsequent analysis of the toxic sequences from the random functional siRNA set revealed that all twelve sequences contained either an AAA/UUU or GCCA/UGGC motif. To determine whether a correlation existed between the motifs and toxicity, three additional, randomly selected, groups of siRNA that contained either AAA/UUU motifs, GCCA/UGGC motifs, or neither motif, were chosen and tested for the ability to induce cell death. Figures 6A-6C are graphical representations that shown the siRNA containing the AAA/UUU and GCCA/UGGC motifs exhibited a higher probability of inducing toxicity (*e.g.*, 56 % and 53 %, respectively) in comparison with the non-motif siRNA (*e.g.,* 6 %). Since the statistical analysis (*e.g.*, T-Test) p-value was $1.3 \times 10^{-7}$ for these two samples, the results show a strong correlation exists between siRNA induced cellular toxicity and delivery of duplexes containing the AAA/UUU or GCCA/UGGC motifs. Thus, siRNA having toxic motifs can be used as transfection controls.

### Example 4

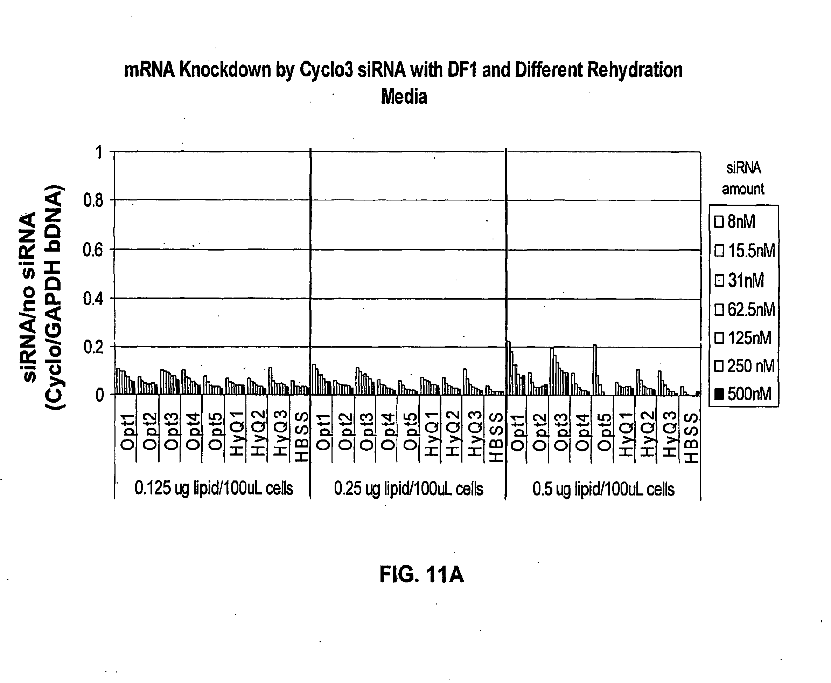

The ability of siRNA having a toxic motif to induce cell death was studied while the RNAi pathway was severely compromised. Previous studies revealed that eIF2C2/hAgo2 is required for mRNA cleavage, and that silencing of these gene products can severely cripple the RNAi pathway. In a control study shown in Figure 11A, a first set of HeLa cells were forward transfected with siRNA directed against eIF2C2 (*e.g.*, siRNA-eIF2C2), and a second set were transfected with control siRNA (*e.g.*, siRNA-RISC-Free) that are not processed by RISC in T1. Each set of the HeLa cells where then transfected with siRNA-RF (i.e., siRNA-RISC-Free) and pEGFP in T2.

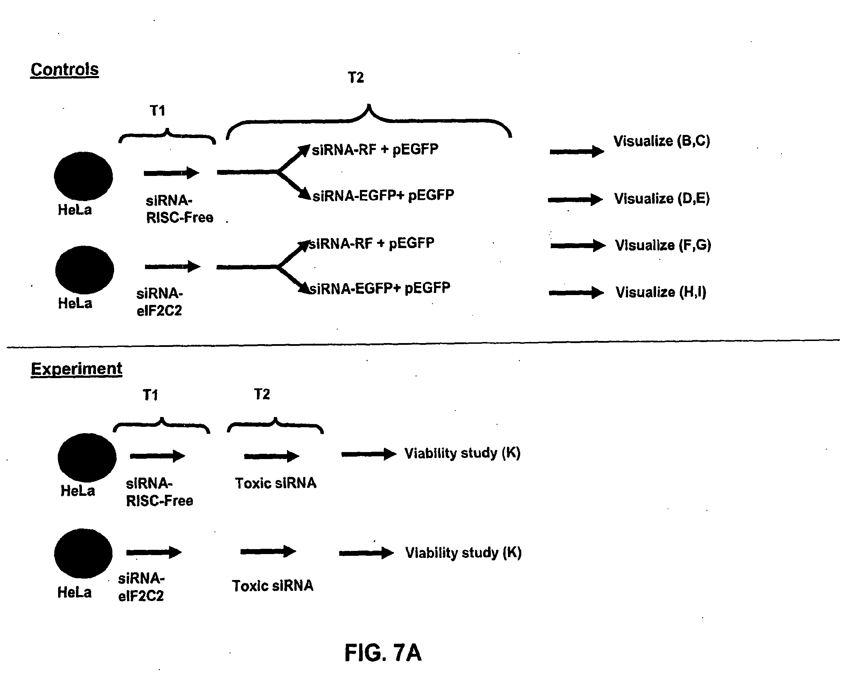

The results of the control study of Figure 7A can be viewed in the images depicted in Figures 7B-7I. The results shown in Figure 7B and 7C show that the siRNA-RISC free does not inhibit EGFP expression from pEGFP. However, Figures 7D and 7E show that the siRNA-EGFP was able to inhibit production of the green fluorescent protein when the RNAi pathway was not compromised. The results in 7F and 7G show that the even if siRNA-eIF2C2 inhibited the functionality of the RNAi pathway, the control siRNA-RF did not have any effect because the cells expressed the green fluorescent protein. Additionally, Figures 7H and 7I show that the siRNA-eIF2C2 inhibited the functionality of the RNAi pathway because the pEGFP show the cells were transfected and expressed the green fluorescent protein in the presence of siRNA-EGFP.

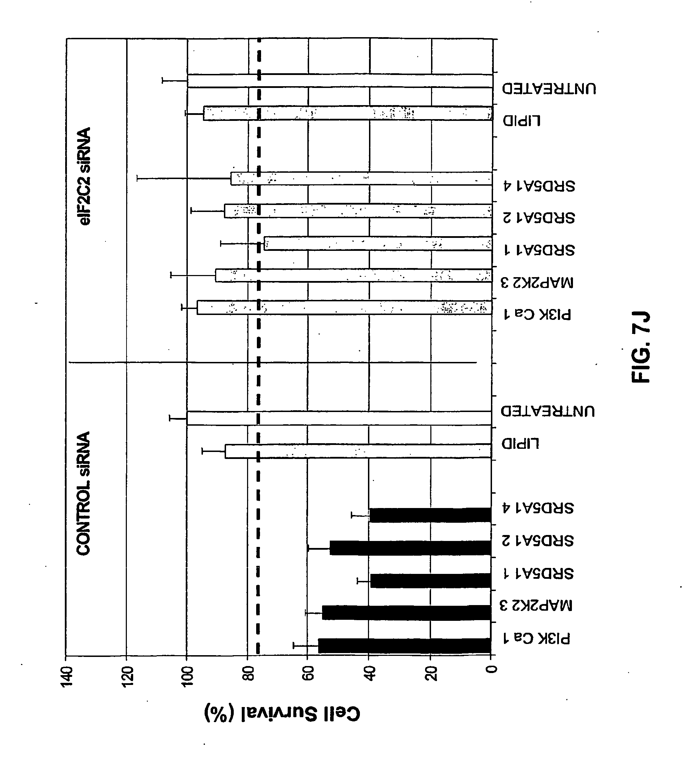

The importance of the RNAi pathway in siRNA-induced cell death was tested by transfecting HeLa with the siRNA-eIF2C2 and control siRNA-RF (T1 of Figure 7A Experiment). The HeLa cells were subsequently transfected with toxic siRNA containing either the AAA/UUU or GCCA/UGG motifs (T2 of Figure 7A Experiment). Figure 7J is a graphical representation of the gene silencing obtained with siRNA-eIF2C2, which eliminated the ability of toxic siRNA to induce cell death. As parallel experiments, cells were pre-transfected with control siRNA-RF (T1) that exhibited toxicity characteristic of these sequences, and it was concluded that an intact RNAi pathway was necessary for siRNA-induced toxicity.

### Example 5

Additional experiments were conducted to determine the involvement of the RNAi pathway in siRNA induced toxicity. The ability of toxic siRNA to induce cell death was tested with siRNA having 19 and 17 base pairs. Previous studies have shown that duplexes that are shorter than 19 base pairs targeted mRNA sequences inefficiently, which suggests that Dicer and/or RISC fail to mediate RNAi when duplex sequence length drops to some level below 19 base pairs.

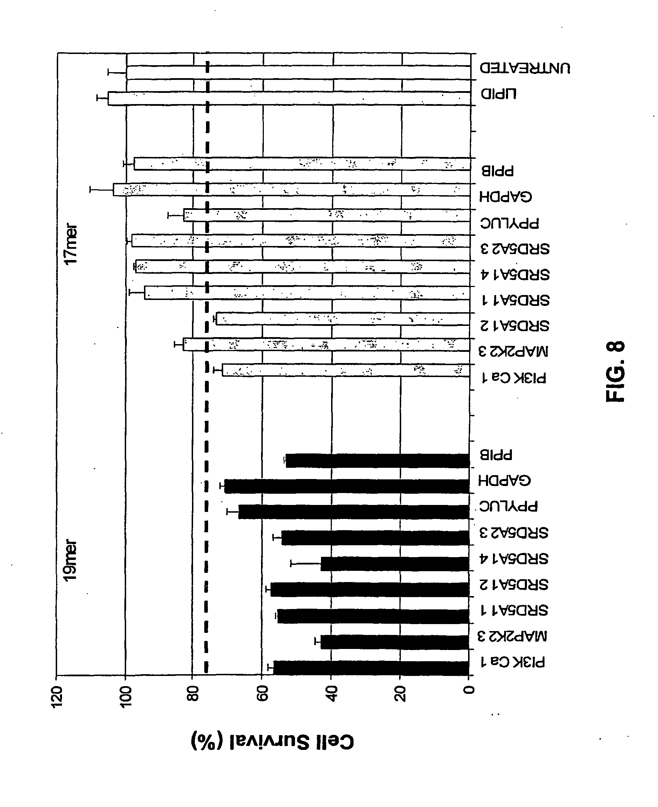

Figure 8 is a graphical representation of the results of toxic siRNA having 19 base pairs and corresponding siRNA having 17 base pairs. As the graph illustrates, the siRNA having 17 base pairs resulted in significantly less toxicity. This suggests that entry and/or processing by RISC is necessary for siRNA to induce toxicity. Thus, preferably the toxic control siRNA is at least 18 base pairs, and more preferably at least 19 base pairs. Together these results demonstrate that siRNA induced off-target effects can generate measurable phenotypes.

### Example 6

Three different lipids were studied on two different cell lines to optimize an RTF protocol. Accordingly, positive control siRNAs (*e.g.*, cyclo3) at various concentrations were dried on well floors of 96-well plates such that final concentrations varied between 0 and 250 nM. Lipid solutions of OLIGOFECTAMINE™, Dharma*FECT*™ 1, or TBio were mixed with Opti-MEM™ and tested on 10,000 A549 cells, or 5,000 3T3L1 cells.

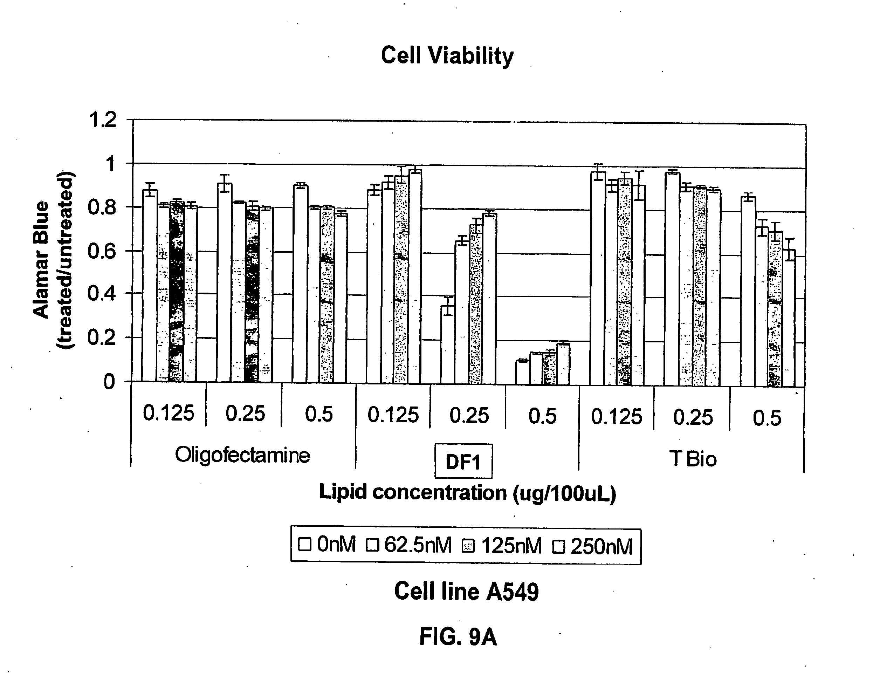

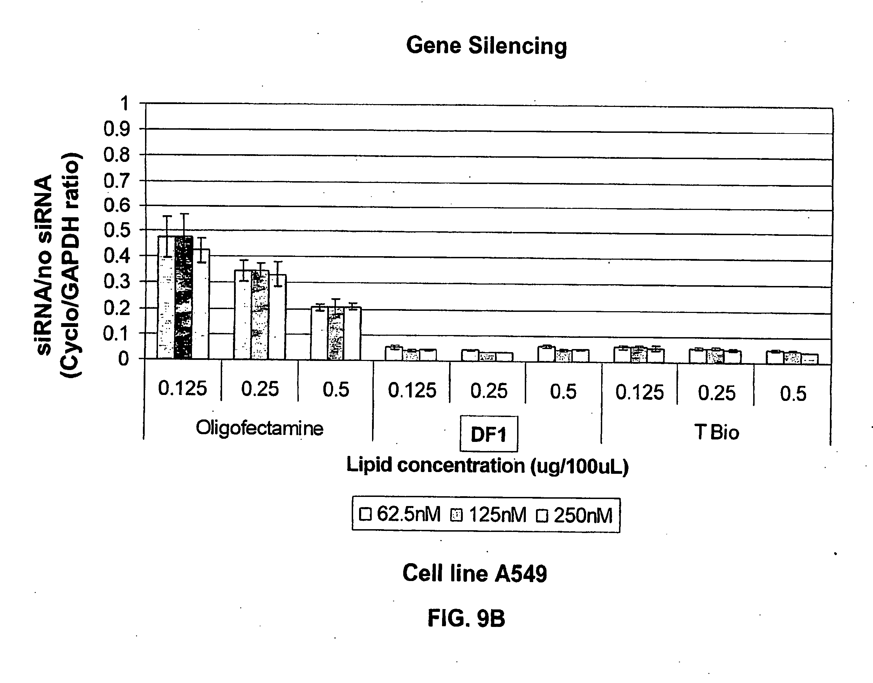

Figure 9A is a graphical representation of the toxicity of the lipid in the A549 cells. OLIGOFECTAMINE™ and TBio induced minimal toxicity at all concentrations. Dharma*FECT*™ 1 produced minimal toxicity at 0.125 ug of lipid per100 uL. Figure 9B is a graphical representation of the gene silencing efficacy achieved with the lipids. OLIGOFECTAMINE™ was shown to have inefficient gene silencing so as to be not suitable for use in siRNA RTF of A549 cells. Dharma*FECT*™ ™1 and TBio provided excellent gene silencing at all amounts of siRNA, and were suitable for use in A549 cells.

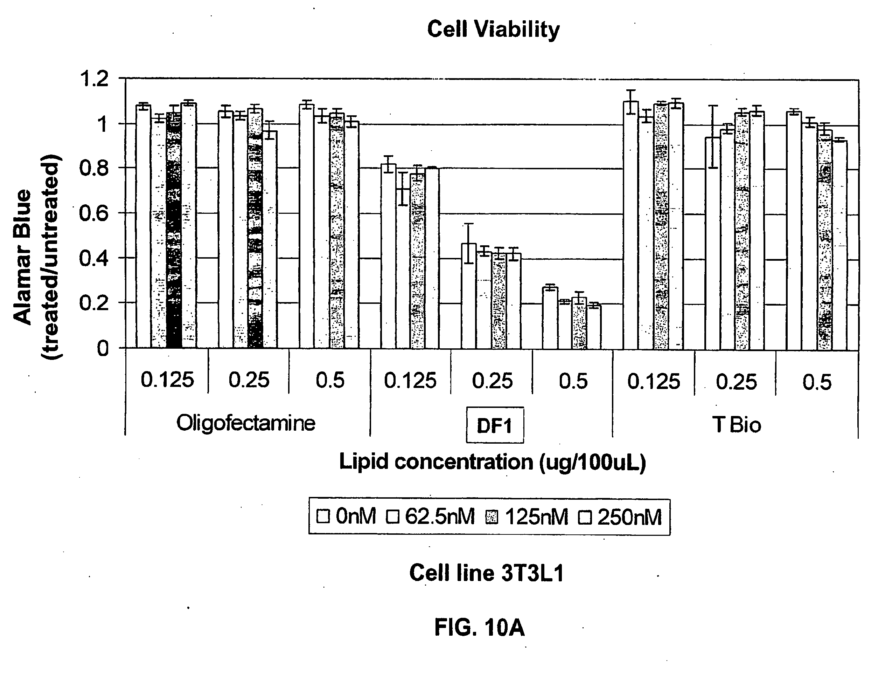

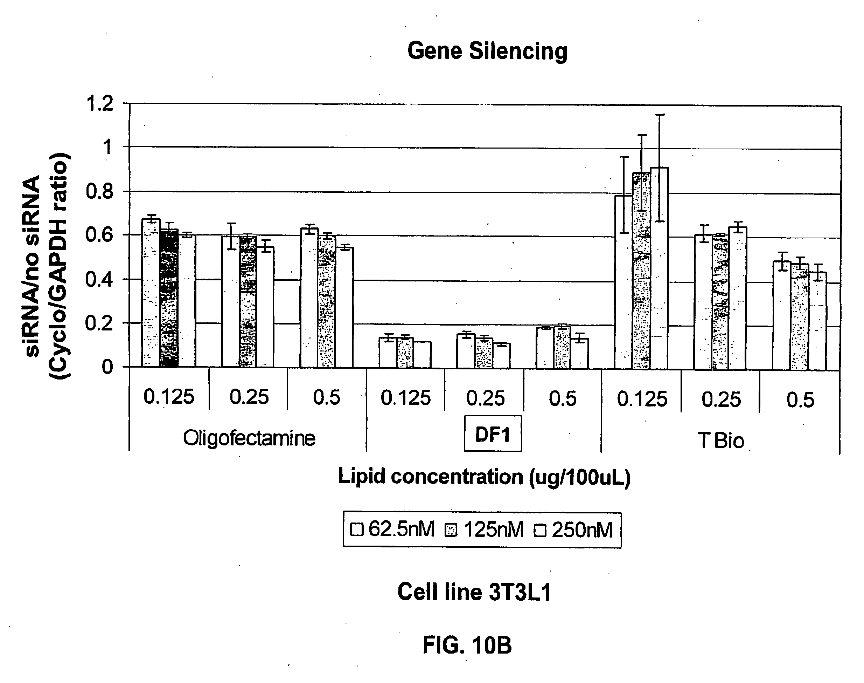

Figure 10A is a graphical representation of the toxicity of the lipid in the 3T3L1 cells. OLIGOFECTAMINE™ and TBio induced minimal toxicity at all concentrations. Dharma*FECT*™ 1 produced minimal toxicity at 0.125 ug of lipid per 100 uL. Figure 10B is a graphical representation of the gene silencing efficacy achieved with the lipids. OLIGOFECTAMINE™ and Tbio each showed inefficient gene silencing so as to be not suitable for use in siRNA RTF of 3T3L1 cells. Dharma*FECT*™ 1 provided excellent gene silencing at all amounts of siRNA, and were suitable for use in 3T3L1 cells.

### Example 7

The optimization of reagents that induce the least amount of cell toxicity and death were studied in a RTF test protocol with three separate lipid-media or lipid-buffer mixtures. Control siRNA (*e.g.*, cyclo3) at various concentrations were used with lipid solutions of Dharma*FECT*™ 1-Opti-MEM™, Dharma*FECT*™ 1-HyQ-MEM™, or Dharma*FECT*™ 1- Hanks Balanced Salt Solution ("HBSS").

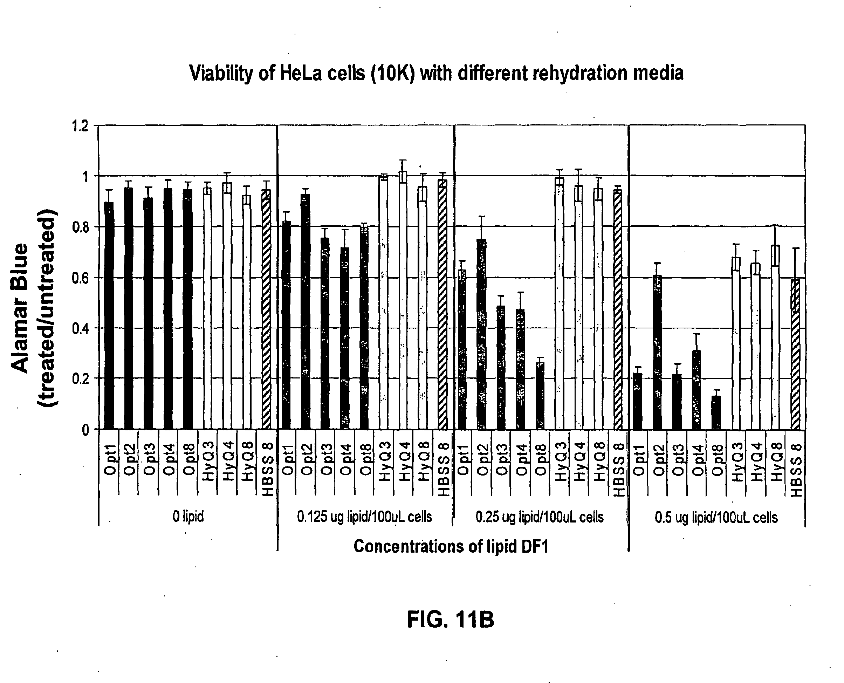

Figure 11A is a graphical representation of the gene silencing obtained with the foregoing lipid solutions. The gene silencing in each culture was shown to be very similar (*e.g.*, >80% silencing. Figure 11B is a graphical representation of the toxicity obtained with the foregoing lipid solutions. The toxicity varied depending on the lipid and media conditions. At 0.125 ug of lipid per 100 uL, both HyQ-MEM™ and HBSS performed more consistently than Opti-MEM™ with nearly 100% cell viability for HyQ-MEM™ and HBSS compared to about 80% viability for Opti-MEM ™. At higher lipid concentrations of 0.25 ug per 100 uL the differences in the performance of Opti-MEM™, HyQ-MEM™ and HBSS are more significant. At 0.25 ug of lipid per 100 uL, cell viability with Dharma*FECT*™ 1-Opti-MEM™ solutions was approximately 60%, 75%, 50%, 50%, and 25% for plates that had been aged 1, 2, 3, 4, and 8 weeks, respectively. In contrast, cell viability of cultures treated with Dharma*FECT*™ 1-HyQ-MEM™ and Dharma*FECT*™ 1-HBSS were greater than 90%. These results identify HyQ-MEM™ and HBSS as preferred reagents for siRNA RTF protocols due to greater consistency across lipid concentrations.

## Example 8

In one example, a RTF testing plate or series of plates can be designed in order to optimize RTF with control siRNA. Accordingly, the plates can be configured to include any of the following variables: (1) the concentration of individual or pools of control siRNA can be between 0.01-250 nM, more preferably between 0.05 and 100 nM, even more preferably between 0.1 and 50 nM, still even more preferably between 0.5 and 25 nM, and most preferably between 0.75 and 10 nM or about 1 nM; (3) the types of polynucleotide carrier can be a lipid such as Dharma*FECT*™ 1, Dharma*FECT*™ 2, Dharma*FECT*™ 3, or Dharma*FECT*™ 4; (3) the concentration of the lipid polynucleotide carrier can be at concentrations of 0.05-1 ug per 100 uL of solution, more preferably at concentrations of 0.05-0.5 ug of lipid per 100 uL of solution, even more preferably still at concentrations of 0.05-0.25 ug of lipid per 100 uL of solution, and most preferably at concentrations of 0.05- 0.1 ug per 100 uL of solution; (4) the types of media and/or buffer used to complex the lipid being preferably Opti-MEM ™, more preferably HyQ-MEM™, and most preferably buffered salt solutions such as Hanks Buffered salt solution or equivalent mixtures; and (5) the types and amounts of cells having densities of 1,000 to 35,000 cells per 0.35 cm$^2$ preferred densities of 2,000-30,000 cells per 0.35 cm$^2$, more preferably 2,000-20,000 cells per 0.35 cm$^2$, even more preferably 2,000-15,000 cells per 0.35 cm$^2$, and most preferably cell densities of 2,000-10,000 cells per 0.35 cm$^2$.

The present invention may be embodied in other specific forms without departing from its essential characteristics. The described embodiments are to be considered in all respects only as illustrative and not restrictive. The scope of the invention is, therefore, indicated by the appended claims rather than by the foregoing description.

## Claims

1. A reverse transfection plate for testing the efficacy of gene silencing, the plate comprising:

   at least a first control well including a dry first control composition having at least a first control siRNA capable of providing a first indication of gene silencing efficacy, the first control composition being devoid of a polynucleotide carrier and configured such that the at least first control siRNA is capable of being solubilized or suspended in an aqueous medium in an amount sufficient for transfecting cells in the first control well, wherein a total amount of control siRNA in the first control composition is present in an amount for transfecting cells in only the first control well.

2. A plate as in claim 1, wherein control siRNA is at least one of a transfection control siRNA, positive control siRNA, or negative control siRNA.

3. A plate as in claim 2, further comprising at least a second control well including a dry second control composition having at least a second control siRNA capable of providing a second indication of gene silencing efficacy that is different from the first indication, the second control composition being configured such that the at least second control siRNA is capable of being solubilized or suspended in an aqueous medium in an amount sufficient for transfecting cells in the second control well.

4. A plate as in claim 2 or 3, wherein the positive control siRNA silences expression of a known gene.

5. A plate as in claim 4, wherein the positive control siRNA silences expression of at least one of a cyclophilin B, lamin A/C, or glyceraldehyde-3-phosphate dehydrogenase.

6. A plate as in claim 2 or 3, wherein the transfection control siRNA includes a label.

7. A plate as in claim 6, wherein the label is coupled with a 5' terminal nucleotide or 3' terminal nucleotide on one of a sense strand or an antisense strand.

8. A plate as in claim 7, wherein the label is a fluorescent label on the sense strand.

9. A plate as in any of claims 2, 3 or 6, wherein the transfection control siRNA is toxic to cells.

10. A plate as in claim 2 or 3, wherein the negative control siRNA is non-functional siRNA.

11. A plate as in claim 10, wherein the non-functional siRNA includes at least one of a 17 base pair duplex containing a 17 base pair duplex having 2' modifications on a first and second sense nucleotide, and having 2' modifications at the first and second antisense nucleotide, or a 19 base pair duplex having 5' deoxy nucleotides on sense and antisense 5' terminal nucleotides.

12. A plate as in claim 3, wherein the first control composition includes a positive control siRNA and the second control composition includes a negative control siRNA.

13. A plate as in claim 3, wherein the first control composition includes a pool of control siRNAs.

14. A plate as in claim 3, further comprising at least a third control well including a dry third control composition having at least a third control siRNA capable of providing a third indication of gene silencing efficacy that is different from at least one of the first indication or second indication, the third control composition being configured such that the at least third control siRNA is capable of being solubilized or suspended in an aqueous medium in an amount sufficient for transfecting cells in the third control well.

15. A plate as in any preceding claim, further comprising at least one well devoid of siRNA.

16. A plate as in any preceding claim, wherein the control siRNA has at least one of a hairpin structure, a stabilizing modification, or conjugate.

17. A plate as in claim 16, wherein the conjugate is selected from the group consisting of amino acids, peptides, polypeptides, proteins, antibodies, lipids, nucleotides, cholesterols and labels.

18. A plate as in claim 17, wherein the conjugate is a cholesterol or other lipid.

19. A plate as in claim 17, wherein the conjugate is a fluorescent label.

20. A plate as in claim 18 or 19, wherein the conjugate is coupled with the 5' terminal nucleotide or 3' terminal nucleotide on one of the sense strand or antisense strand.

21. A plate as in claim 18 or 19, wherein the conjugate is linked through a linker selected from the group consisting of modified nucleotides, unmodified nucleotides, polyethers, polyethylene glycols, polyalcohols, polypropylenes, polyalkylamines, polyamines, spermidine, polyesters, polyethyl acrylate, polyphosphodiesters, carbohydrates, sugars, propylene glycols, ethylene glycols, alkylenes, and combinations thereof.

22. A kit comprising a plate as claimed in any preceding claim in combination with a polynucleotide carrier configured to complex with the at least first control siRNA and an aqueous medium, the combination to serve as a reverse transfection system for testing the efficacy of gene silencing.

23. A method of testing the efficacy of gene silencing of an siRNA with control siRNA, the method comprising:

   providing a well plate having a plurality of wells including:

at least a first control well including a dry first control composition having at least a first control siRNA capable of providing a first indication of gene silencing efficacy, the dry first control composition having been dried in the first control well without a polynucleotide carrier and the dry first control composition being configured such that the at least first control siRNA is capable of being solubilized or suspended in an aqueous medium in an amount sufficient for transfecting cells in the first control well, and wherein a total amount of control siRNA in the first control composition is present in an amount for transfecting cells in only the first control well;

adding an aqueous medium to the first control well;

solubilizing or suspending the dry first control composition in the aqueous medium;

adding cells to the first control well under conditions that permit transfection; and

determining the effect of the first control siRNA on the cells.

24. A method as in claim 23, wherein the aqueous medium includes a polynucleotide carrier.

25. A method as in claim 23 or 24, wherein the control siRNA is **characterized by** at least one of the following:

a positive control siRNA that silences expression of a known gene;

a transfection control siRNA that includes a fluorescent label;

a transfection control siRNA that is toxic to cells;

a negative control siRNA that is non-functional siRNA; or

a negative control siRNA configured to inhibit being taken in and processed by RISC.

26. A method as in claim 25, further comprising:

adding the aqueous medium to a second control well in the well plate, wherein the second control well includes a second control siRNA;

adding cells to the second control well under conditions that permit transfection; and

determining the effect of the second control siRNA on the cells.

27. A method as in claim 26, further comprising:

adding the aqueous medium to a third control well in the well plate, wherein the third control well includes a third control siRNA;

adding cells to the third control well under conditions that permit transfection; and

determining the effect of the third control siRNA on the cells.

28. A method as in claim 25, further comprising:

adding an aqueous medium to a blank well in the well plate, the blank well being devoid of siRNA;

adding cells to the blank well; and

comparing the effect of the first control siRNA on the cells in the first control well with the cells added to the blank well.

29. A method as in claim 25, further comprising:

comparing the effect of the first control siRNA on the cells with a known effect of the first control siRNA.

30. A method as in claim 24, wherein the polynucleotide carrier is a lipid.

31. A method as in any of claims 23 to 30, wherein the cells are added in an amount of about $2 \times 10^3$ to about $3 \times 10^4$ cells per about 0.30 cm$^2$ to about 0.35 cm$^2$ of cell growth surface area.

32. A method for making a reverse transfection plate for testing the efficacy of gene silencing, the method comprising:

providing a well plate having a plurality of wells including a first control well;

providing a first control siRNA capable of providing a first indication of gene silencing efficacy and solubilising the first control siRNA without a polynucleotide carrier;

applying the solubilised control siRNA solution without polynucleotide carrier to the floor of the first control well in an amount sufficient for transfecting the cells in the first control well, wherein a total amount of control siRNA in the first control composition is present in an amount for transfecting cells in only the first control well; and drying the control siRNA on the plate.

33. A method as in claim 32, wherein control siRNA is at least one of a transfection control siRNA, positive control siRNA, or negative control siRNA.

34. A method as in claim 33, wherein the transfection control siRNA includes a label.

35. A method as in any of claims 32 to 34, wherein the control siRNA has at least one of a hairpin structure, a stabilizing modification, or conjugate.

36. A method as in claim 35, wherein the conjugate is a cholesterol or other lipid.

37. A method as in claim 35, wherein the conjugate is a fluorescent label.


**Patentansprüche**

1. Platte für die reverse Konstruktion zum Testen der Wirksamkeit der Gen-Stilllegung, wobei die Platte Folgendes umfasst:

zumindest eine erste Kontroll-Probenschale, die eine trockene erste Kontroll-Zusammensetzung mit zumindest einer Kontroll-siRNA einschließt, die eine erste Anzeige einer Wirksamkeit der Gen-Stilllegung ergibt, wobei die erste Kontroll-Zusammensetzung frei von einem Polynukleotid-Träger ist und so konfiguriert ist, dass die zumindest erste Kontroll-siRNA in der Lage ist, in einem wässrigen Medium in einer Menge lösbar gemacht oder suspendiert zu werden, die ausreicht, um Zellen in der ersten Kontroll-Probenschale zu transfizieren, wobei eine Gesamtmenge an Kontroll-siRNA in der ersten Kontroll-Zusammensetzung in einer Menge zum Transfizieren von Zellen in lediglich der ersten Kontroll-Probenschale vorliegt.

2. Platte nach Anspruch 1, bei der die Kontroll-siRNA zumindest eine von einer Transfektions-Kontroll-siRNA, einer positiven Kontroll-siRNA oder einer negativen Kontroll-siRNA ist.

3. Platte nach Anspruch 1, die zumindest eine zweite Kontroll-Probenschale umfasst, die eine trockene zweite Kontroll-Zusammensetzung einschließt, die zumindest eine zweite Kontroll-siRNA aufweist, die in der Lage ist, eine zweite Anzeige einer Wirksamkeit der Gen-Stilllegung zu liefern, die von der ersten Anzeige verschieden ist, wobei die zweite Kontroll-Zusammensetzung so konfiguriert ist, dass die zumindest zweite Kontroll-siRNA in der Lage ist, lösbar gemacht oder in einem wässrigen Medium in einer Menge suspendiert zu werden, die zur Transfektion von Zellen in der zweiten Kontroll-Probenschale ausreicht.

4. Platte nach Anspruch 2 oder 3, bei der die positive Kontroll-siRNA die Expression eines bekannten Gens stilllegt.

5. Platte nach Anspruch 4, bei der die positive Kontroll-siRNA die Expression von zumindest einem von Cyclophilin B, Lamin A/C oder Glyceraldehyd-3-Phosphat-Dehydrogenase stilllegt.

6. Platte nach Anspruch 2 oder 3, bei der die Transfektions-Kontroll-siRNA ein Label einschließt.

7. Platte nach Anspruch 6, bei der die Markierung mit einem 5'-End-Nukleotid oder einem 3'-End-Nukleotid an einem von einem codierenden Strang oder einem nicht-codierenden Strang gekoppelt ist.

8. Platte nach Anspruch 7, bei der die Markierung eine fluoreszierende Markierung auf dem Sense-Strand ist.

9. Platte nach einem der Ansprüche 2, 3 oder 6, bei der Transfektions-Kontroll-siRNA für Zellen toxisch ist.

10. Platte nach Anspruch 2 oder 3, bei der die negative Kontroll-siRNA eine nicht funktionelle siRNA ist.

11. Platte nach Anspruch 10, bei der die nicht funktionelle siRNA zumindest eines von einem 17-Basis-Paar-Duplex, das ein 17-Basis-Paar-Duplex mit 2'-Modifikationen auf einem ersten und zweiten codierenden Nukleotid enthält und 2'-Modifikationen an dem ersten und zweiten codierenden Nukleotid hat, oder ein 19-Basis-Paar-Duplex einschließt, das 5'-Deoxy-Nukleotide auf codierenden und nicht-codierenden 5'-End-Nukleotiden hat.

12. Platte nach Anspruch 3, bei der die erste Kontroll-Zusammensetzung eine positive Kontroll-siRNA einschließt und die zweite Kontroll-Zusammensetzung eine negative Kontroll-siRNA einschließt.

13. Platte nach Anspruch 3, bei der die erste Kontroll-Zusammensetzung einen Pool von Kontroll-siRNAs einschließt.

14. Platte nach Anspruch 3, die weiterhin zumindest eine dritte Kontroll-Probenschale umfasst, die eine trockene dritte Kontroll-Zusammensetzung mit zumindest einer dritten Kontroll-siRNA aufweist, die in der Lage ist, eine dritte Anzeige der Wirksamkeit einer Gen-Stilllegung zu liefern, die von zumindest einer der ersten Anzeigen und der zweiten Anzeigen verschieden ist, wobei die dritte Kontroll-Zusammensetzung so konfiguriert ist, dass die zumindest dritte Kontroll-siRNA in der Lage ist, in einem wässrigen Medium lösbar gemacht oder suspendiert zu werden, und zwar in einer Menge, die ausreicht, um Zellen in der dritten Kontroll-Probenschale zu transfizieren.

15. Platte nach einem der vorhergehenden Ansprüche, die weiterhin zumindest eine von siRNA freie Probenschale umfasst.

16. Platte nach einem der vorhergehenden Ansprüche, bei der die Kontroll-siRNA zumindest eines von einer Haarnadel-Struktur, einer Stabilisierungs-Modifikation oder einem Konjugat hat.

17. Platte nach Anspruch 16, bei der das Konjugat aus der Gruppe ausgewählt ist, die aus Aminosäuren, Peptiden, Polypeptiden, Proteinen, Antikörpern, Lipiden, Nukleotiden, Cholesterolen und Markierungen besteht.

18. Platte nach Anspruch 17, bei der das Konjugat ein Cholesterol oder ein anderes Lipid ist.

19. Platte nach Anspruch 17, bei der das Konjugat ein Fluoreszenz-Label ist.

20. Platte nach Anspruch 18 oder 19, bei der das Konjugat mit dem 5'-End-Nukleotid oder dem 3'-End-Nukleotid auf einen von dem codierenden Strang oder dem nicht-codierenden Strang gekoppelt ist.

21. Platte nach Anspruch 18 oder 19, bei der das Konjugat über ein Link-Element verbunden ist, das aus der Gruppe ausgewählt ist, das aus modifizierten Nukleotiden, nicht-modifizierten Nukleotiden, Polyethern, Polyethylenglycolen, Polyalkoholen, Polypropylenen, Polyalkyaminen, Polyaminen, Spermidinen, Polyestern, Polyethylenacrylaten, Polyphosphodiestern, Karbohydraten, Zuckern, Propylenglycolen, Ethylenglycolen, Alkylenen und Kombinationen hiervon besteht.

22. Ein Kit, das eine Platte nach einem der vorhergehenden Ansprüche, in Kombination mit einem Polynukleotid-Träger umfasst, der so konfiguriert ist, dass er komplex zu der zumindest ersten Kontroll-siRNA und einem wässrigen Medium einen Komplex bildet, wobei die Kombination als eine Revers-Transfektions-System zum Testen der Wirksamkeit einer Gen-Stilllegung konfiguriert ist.

23. Verfahren zum Testen der Wirksamkeit einer Gen-Stilllegung von siRNA mit einer Kontroll-siRNA, wobei das Verfahren Folgendes umfasst:

Bereitstellen einer Probenschalen-Platte mit einer Anzahl von Probenschalen, die Folgendes einschließen:

zumindest eine erste Kontroll-Probenschale, die eine trockene erste Kontroll-Zusammensetzung mit zumindest einer ersten Kontroll-siRNA einschließt, die in der Lage ist, eine erste Anzeige der Wirksamkeit der Gen-Stilllegung liefert, wobei die trockene Kontroll-Zusammensetzung in der ersten Kontroll-Probenschale ohne einen Polynukleotid-Träger getrocknet wurde und die trockene Kontroll-Zusammensetzung derart konfiguriert ist, dass die zumindest erste Kontroll-siRNA in der Lage ist, in einem wässrigen Medium in einer Menge lösbar gemacht oder suspendiert zu werden, die ausreicht, um Zellen in der ersten Kontroll-Probenschale zu transfizieren, und bei dem die Gesamtmenge an Kontroll-siRNA in der ersten Kontroll-Zusammensetzung in einer Menge zum Transfizieren von Zellen in lediglich der ersten Kontroll-Zelle vorliegt;
Hinzufügen eines wässrigen Mediums zu der ersten Kontroll-Probenschale;
Lösbarmachen oder suspendieren der trockenen ersten Kontroll-Zusammensetzung in der wässrigen Lösung;
Hinzufügen von Zellen zu der ersten Kontroll-Probenschale unter Bedingungen, die eine Transfektion ermöglichen; und
Bestimmen der Wirkung der ersten Kontroll-siRNA auf die Zellen.

24. Verfahren nach Anspruch 23, bei dem das wässrige Medium einen Polynukleotid-Träger einschließt.

25. Verfahren nach Anspruch 23 oder 24, bei dem die Kontroll-siRNA durch zumindest eines der Folgenden charakterisiert ist:

eine positive Kontroll-siRNA, die die Expression eines bekannten Gens stilllegt;
eine Transfektions-Kontroll-siRNA, die ein fluoreszierendes Label einschließt,
eine Transfektions-Kontroll-siRNA, die gegenüber Zellen toxisch ist;
eine negative Kontroll-siRNA, die eine nicht funktionelle siRNA ist, oder eine negative Kontroll-siRNA, die so konfiguriert ist, dass sie die Aufnahme in RISC hindert und durch dieses verarbeitet wird.

26. Verfahren nach Anspruch 25, das weiterhin Folgendes umfasst:

Hinzufügen des wässrigen Mediums zu einer zweiten Kontroll-Probenschale in der Probenschalenplatte, wobei die zweite Kontroll-Probenschale eine zweite Kontroll-siRNA einschließt;
wobei die zweite Kontroll-Probenschale eine zweite Kontroll-siRNA einschließt;
Hinzufügen von Zellen zu der zweiten Kontroll-Zelle unter Bedingungen, die eine Transfektion ermöglichen; und
Bestimmen der Wirkung der zweiten Kontroll-siRNA auf die Zellen.

27. Verfahren nach Anspruch 26, das weiterhin Folgendes umfasst:

Hinzufügen des wässrigen Mediums zu einer dritten Kontroll-Probenschale in der Probenschalenplatte, wobei die dritte Kontroll-Probenschale eine dritte Kontroll-siRNA einschließt;
Hinzufügen von Zellen zu der dritten Kontroll-Probenschale unter Bedingungen, die eine Transfektion ermöglichen; und
Bestimmen der Wirkung der dritten Kontroll-siRNA auf die Zellen.

28. Verfahren nach Anspruch 25, das weiterhin Folgendes umfasst:

Hinzufügen eines wässrigen Mediums zu einer rohen Probenschale in der Probenschalenplatte, wobei die rohe Probenschale frei von siRNA ist;
Hinzufügen von Zellen zu der leeren Probenschale; und
Vergleichen der Wirkung der ersten Kontroll-siRNA auf die Zellen in der ersten Kontroll-Probenschale mit den Zellen, die zu der leeren Probenschale hinzugefügt werden.

29. Verfahren nach Anspruch 25, das weiterhin Folgendes umfasst:

Vergleichen der Wirkung der ersten Kontroll-siRNA auf die Zellen mit einem bekannten Effekt der ersten Kontroll-siRNA umfasst.

30. Verfahren nach Anspruch 24, bei dem der Polynukleotid-Träger ein Lipid ist.

31. Verfahren nach einem der Ansprüche 23 bis 30, bei dem die Zellen in einer Menge von ungefähr $2 \times 10^3$ bis ungefähr $3 \times 10^4$ Zellen pro ungefähr $0{,}30 \ cm^2$ bis ungefähr $0{,}35 \ cm^2$ an Zellenwachstum-Oberfläche hinzugefügt werden.

32. Verfahren zur Herstellung einer Revers-Transfektions-Platte zum Testen der Wirksamkeit der Gen-Stilllegung, wobei das Verfahren Folgendes umfasst:

Bereitstellen einer Probenschalenplatte mit einer Anzahl von Probenschalen, die eine erste Kontroll-Probenschale einschließen;
Bereitstellen einer ersten Kontroll-siRNA, die in der Lage ist, eine erste Anzeige der Gen-Stilllegung und Lösbarkeit der ersten Kontroll-siRNA ohne einen Polynukleotid-Träger zu liefern;
Anwenden der lösbar gemachten Kontroll-siRNA-Lösung ohne einen Polynukleotid-Träger auf dem Boden der ersten Kontroll-Probenschale in einer Menge, die ausreicht, um die Zellen in der ersten Kontroll-Probenschale zu transfizieren, wobei die Gesamtmenge der Kontroll-siRNA in der ersten Kontroll-Zusammensetzung in einer Menge zum Transfizieren von Zellen in lediglich der ersten Kontroll-Probenschale vorliegt; und Trocknen der Kontroll-siRNA auf der Platte.

**33.** Verfahren nach Anspruch 32, bei dem die Kontroll-siRNA zumindest eine von einer Transfektions-Kontroll-siRNA, einer positiven Kontroll-siRNA oder einer negativen Kontroll-siRNA ist.

**34.** Verfahren nach Anspruch 31, bei dem die Transfektions-Kontroll-siRNA ein Label einschließt.

**35.** Verfahren nach einem der Ansprüche 32 bis 34, bei dem die Kontroll-siRNA zumindest eine von einer Haarnadel-Struktur, einer Stabilisierungs-Modifikation oder einem Konjugat hat.

**36.** Verfahren nach Anspruch 35, bei dem das Konjugat ein Cholesterol oder anderes Lipid ist.

**37.** Verfahren nach Anspruch 35, bei dem das Konjugat eine fluoreszente Markierung ist.

**Revendications**

**1.** Plaque de transfection inverse pour analyser l'efficacité de la réduction au silence de gènes, la plaque comprenant :

au moins un premier puits contrôle comprenant une première composition contrôle sèche ayant au moins un premier siRNA contrôle capable de fournir une première indication de l'efficacité de la réduction au silence de gènes, la première composition contrôle étant dépourvue de support polynucléotidique et configurée de telle manière qu'au moins le premier siRNA contrôle est capable d'être solubilisé ou mis en suspension dans un milieu aqueux dans une quantité suffisante pour transfecter les cellules dans le premier puits contrôle, où une quantité totale de siRNA contrôle dans la première composition contrôle est présente dans une quantité pour transfecter les cellules dans le premier puits contrôle uniquement.

**2.** Plaque selon la revendication 1, dans laquelle le siRNA contrôle est au moins l'un d'un siRNA contrôle de transfection, d'un siRNA contrôle positif ou d'un siRNA contrôle négatif.

**3.** Plaque selon la revendication 2, comprenant en outre au moins un deuxième puits contrôle comprenant une deuxième composition contrôle sèche ayant au moins un deuxième siRNA contrôle capable de fournir une deuxième indication de l'efficacité de la réduction au silence de gènes qui est différente de la première indication, la deuxième composition contrôle étant configurée de telle manière qu'au moins le deuxième siRNA contrôle est capable d'être solubilisé ou mis en suspension dans un milieu aqueux dans une quantité suffisante pour transfecter les cellules dans le deuxième puits contrôle.

**4.** Plaque selon la revendication 2 ou 3, dans laquelle le siRNA contrôle positif réduit au silence l'expression d'un gène connu.

**5.** Plaque selon la revendication 4, dans laquelle le siRNA contrôle positif réduit au silence l'expression d'au moins l'une d'une cyclophiline B, d'une lamine A/C ou d'une glycéraldéhyde-3-phosphate déshydrogénase.

**6.** Plaque selon la revendication 2 ou 3, dans laquelle le siRNA contrôle de transfection comprend un marqueur.

**7.** Plaque selon la revendication 6, dans laquelle le marqueur est couplé avec un nucléotide terminal en 5' ou un nucléotide terminal en 3' sur l'un d'un brin sens ou d'un brin antisens.

**8.** Plaque selon la revendication 7, dans laquelle le marqueur est un marqueur fluorescent sur le brin sens.

**9.** Plaque selon l'une quelconque des revendications 2, 3 ou 6, dans laquelle le siRNA contrôle de transfection est toxique pour les cellules.

**10.** Plaque selon la revendication 2 ou 3, dans laquelle le siRNA contrôle négatif est un siRNA non fonctionnel.

**11.** Plaque selon la revendication 10, dans laquelle le siRNA non fonctionnel comprend au moins l'un d'un duplex de 17 paires de bases contenant un duplex de 17 paires de bases présentant des modifications en 2' sur un premier et un deuxième nucléotide sens, et présentant des modifications en 2' au niveau du premier et du deuxième nucléotide antisens, ou d'un duplex de 19 paires de bases présentant des désoxynucléotides en 5' sur les nucléotides terminaux en 5' sens et antisens.

**12.** Plaque selon la revendication 3, dans laquelle la première composition contrôle comprend un siRNA contrôle positif et la deuxième composition contrôle comprend un siRNA contrôle négatif.

**13.** Plaque selon la revendication 3, dans laquelle la première composition contrôle comprend un groupe de siRNA contrôles.

**14.** Plaque selon la revendication 3, comprenant en outre au moins un troisième puits contrôle comprenant une troisième composition contrôle sèche ayant au moins un troisième siRNA contrôle capable de fournir une troisième indication de l'efficacité de la réduction au silence de gènes qui est différente d'au moins l'une de la première indication ou de la deuxième indication, la troisième composition contrôle étant configurée de telle manière qu'au moins le troisième siRNA contrôle est capable d'être solubilisé ou mis en suspension dans un milieu aqueux dans une quantité suffisante pour transfecter les cellules dans le troisième puits contrôle.

**15.** Plaque selon l'une quelconque des revendications précédentes, comprenant en outre au moins un puits dépourvu de siRNA.

**16.** Plaque selon l'une quelconque des revendications précédentes, dans laquelle le siRNA contrôle présente au moins l'un d'une structure en épingle à cheveux, d'une modification stabilisante ou d'un conjugué.

**17.** Plaque selon la revendication 16, dans laquelle le conjugué est choisi dans le groupe comprenant des acides aminés, des peptides, des polypeptides, des protéines, des anticorps, des lipides, des nucléotides, des cholestérols et des marqueurs.

**18.** Plaque selon la revendication 17, dans laquelle le conjugué est un cholestérol ou un autre lipide.

**19.** Plaque selon la revendication 17, dans laquelle le conjugué est un marqueur fluorescent.

**20.** Plaque selon la revendication 18 ou 19, dans laquelle le conjugué est couplé au nucléotide terminal en 5' ou au nucléotide terminal en 3' sur l'un du brin sens ou du brin antisens.

**21.** Plaque selon la revendication 18 ou 19, dans laquelle le conjugué est lié par l'intermédiaire d'un lieur choisi dans le groupe consistant en des nucléotides modifiés, des nucléotides non modifiés, des polyéthers, des polyéthylène glycols, des polyalcools, des polypropylènes, des polyalkylamines, des polyamines, la spermidine, des polyesters, un polyacrylate d'éthyle, des polyphosphodiesters, des glucides, des sucres, des propylène glycols, des éthylène glycols, des alkylènes et des combinaisons de ceux-ci.

**22.** Kit comprenant une plaque selon l'une quelconque des revendications précédentes en combinaison avec un support polynucléotidique configuré pour se complexer avec au moins le premier siRNA contrôle et un milieu aqueux, la combinaison pour servir de système de transfection inverse pour analyser l'efficacité de la réduction au silence de gènes.

**23.** Procédé d'analyse de l'efficacité de la réduction au silence de gènes d'un siRNA avec un siRNA contrôle, le procédé comprenant :

la fourniture d'une plaque de puits présentant une pluralité de puits y compris :

au moins un premier puits contrôle comprenant une première composition contrôle sèche ayant au moins un premier siRNA contrôle capable de fournir une première indication de l'efficacité de la réduction au silence de gènes, la première composition contrôle sèche ayant été séchée dans le premier puits contrôle sans support polynucléotidique et la première composition contrôle sèche étant configurée de telle manière qu'au moins le premier siRNA contrôle est capable d'être solubilisé ou mis en suspension dans un milieu aqueux dans une quantité suffisante pour transfecter les cellules dans le premier puits contrôle, et où une quantité totale de siRNA contrôle dans la première composition contrôle est présente dans une quantité pour transfecter les cellules dans le premier puits contrôle uniquement ;

l'addition d'un milieu aqueux dans le premier puits contrôle ;

la solubilisation ou la mise en suspension de la première composition contrôle sèche dans le milieu aqueux ;

l'addition des cellules au premier puits contrôle dans des conditions qui permettent une transfection ; et

la détermination de l'effet du premier siRNA contrôle sur les cellules.

24. Procédé selon la revendication 23, dans lequel le milieu aqueux comprend un support polynucléotidique.

25. Procédé selon la revendication 23 ou 24, dans lequel le siRNA contrôle est **caractérisé par** au moins l'une des propriétés suivantes :

un siRNA contrôle positif qui réduit au silence l'expression d'un gène connu ;

un siRNA contrôle de transfection qui comprend un marqueur fluorescent ;

un siRNA contrôle de transfection qui est toxique pour les cellules ;

un siRNA contrôle négatif qui est un siRNA non fonctionnel ; ou

un siRNA contrôle négatif configuré pour inhiber le fait d'être pris dans et traité par un RISC.

26. Procédé selon la revendication 25, comprenant en outre :

l'addition du milieu aqueux dans un deuxième puits contrôle dans la plaque de puits, dans laquelle le deuxième puits contrôle comprend un deuxième siRNA contrôle ;

l'addition des cellules au deuxième puits contrôle dans des conditions qui permettent une transfection ; et

la détermination de l'effet du deuxième siRNA contrôle sur les cellules.

27. Procédé selon la revendication 26, comprenant en outre :

l'addition du milieu aqueux à un troisième puits contrôle dans la plaque de puits, dans laquelle le troisième puits contrôle comprend un troisième siRNA contrôle ;

l'addition des cellules au troisième puits contrôle dans des conditions qui permettent une transfection ; et

la détermination de l'effet du troisième siRNA contrôle sur les cellules.

28. Procédé selon la revendication 25, comprenant en outre :

l'addition d'un milieu aqueux à un puits de blanc dans la plaque de puits, le puits de blanc étant dépourvu de siRNA ;

l'addition de cellules au puits de blanc ; et

la comparaison de l'effet du premier siRNA contrôle sur les cellules dans le premier puits contrôle aux cellules ajoutées dans le puits de blanc.

29. Procédé selon la revendication 25, comprenant en outre :

la comparaison de l'effet du premier siRNA contrôle sur les cellules à un effet connu du premier siRNA contrôle.

30. Procédé selon la revendication 24, dans lequel le support polynucléotidique est un lipide.

31. Procédé selon l'une quelconque des revendications 23 à 30, dans lequel les cellules sont ajoutées dans une quantité d'environ $2 \times 10^3$ à environ $3 \times 10^4$ cellules pour environ $0,30 \ cm^2$ à environ $0,35 \ cm^2$ de surface spécifique de croissance cellulaire.

32. Procédé de fabrication d'une plaque de transfection inverse pour l'analyse de l'efficacité de la réduction au silence de gènes, le procédé comprenant :

la fourniture d'une plaque de puits présentant une pluralité de puits y compris un premier puits contrôle ;

la fourniture d'un premier siRNA contrôle capable de fournir une première indication de l'efficacité de la réduction au silence de gènes et la solubilisation du premier siRNA contrôle sans support polynucléotidique ;

l'application de la solution de siRNA contrôle solubilisé sans support polynucléotidique sur le fond du premier puits contrôle dans une quantité suffisante pour transfecter les cellules dans le premier puits contrôle, où une quantité totale de siRNA contrôle dans la première composition contrôle est présente dans une quantité pour transfecter des cellules dans le premier puits contrôle uniquement ; et
le séchage du siRNA contrôle sur la plaque.

33. Procédé selon la revendication 32, dans lequel le siRNA contrôle est au moins l'un d'un siRNA contrôle de transfection, d'un siRNA contrôle positif ou d'un siRNA contrôle négatif.

34. Procédé selon la revendication 33, dans lequel le siRNA contrôle de transfection comprend un marqueur.

35. Procédé selon l'une quelconque des revendications 32 à 34, dans lequel le siRNA contrôle présente au moins l'un d'une structure en épingle à cheveux, d'une modification stabilisante ou d'un conjugué.

36. Procédé selon la revendication 35, dans lequel le conjugué est un cholestérol ou un autre lipide.

37. Procédé selon la revendication 35, dans lequel le conjugué est un marqueur fluorescent.

FIG. 1A

| Tc1 | + | P1 | 1A | 1B | 1C | $1_N$ |
| Tc2 | + | P1 | 2A | 2B | 2C | $2_N$ |
| Tc3 | + | $P1_N$ | 3A | 3B | 3C | $3_N$ |
| | - | P2 | 4A | 4B | 4C | $4_N$ |
| | - | P2 | 5A | 5B | 5C | $5_N$ |
| | - | $P2_N$ | 6A | 6B | 6C | $6_N$ |

FIG. 1B

|   | 1 | 2 | 3 | 4 | 5 | 6 | 7 | 8 | 9 | 10 | 11 | 12 |
|---|---|---|---|---|---|---|---|---|---|----|----|----|
| A |   |   |  ← si*CONTROL* Non-Targeting siRNA pool | | | | | | | | | |
| B |   |   |  ← si*CONTROL* RISC-Free siRNA | | | | | | | | | |
| C |   |   |  ← si*GLO* RISC-Free siRNA | | | | | | | | | |
| D |   |   |  ← GAPDH *SMART*pool siRNA | | | | | | | | | |
| E |   |   |  ← si*CONTROL* Cyclophilin B siRNA | | | | | | | | | |
| F |   |   |  ← si*CONTROL* Lamin A/C siRNA or User defined control siRNA | | | | | | | | | |
| G |   |   |  ← Dharma*FECT* transfection reagent | | | | | | | | | |
| H |   |   |  ← Untreated | | | | | | | | | |

☐ No siRNA          ☐ Positive Control siRNAs

☐ Negative Control siRNAs    *SMART*pool siRNA Reagents

**FIG. 2A**

FIG. 2B

FIG. 2C

**FIG. 2D**

**PLAIN PLATES**

siRNA/no siRNA (Cyclo/Gapdh ratio)

□ 62.5nM □ 125nM ■ 250nM

Lipid concentrations (ug/100uL)

Lipofectamine 2000

0.125   0.25   0.5

**KNOCKDOWN**

**FIG. 3B**

**PLAIN PLATES**

Alamar Blue (treated/untreated)

□ 0nM □ 62.5nM □ 125nM ■ 250nM

Lipid concentrations (ug/100uL)

Lipofectamine 2000

0.125   0.25   0.5

**VIABILITY**

**FIG. 3A**

POLY L-LYSINE PLATES

POLY L-LYSINE PLATES

VIABILITY

KNOCKDOWN

FIG. 3C

FIG. 3D

EP 1 814 895 B1

**CELLBIND PLATES**

**CELLBIND PLATES**

**VIABILITY**

**KNOCKDOWN**

**FIG. 3E**

**FIG. 3F**

**VIABILITY**

**FIG. 3G**

**KNOCKDOWN**

**FIG. 3H**

EP 1 814 895 B1

FIBRONECTIN PLATES

VIABILITY

FIG. 3I

FIBRONECTIN PLATES

KNOCKDOWN

FIG. 3J

FIG. 4

FIG. 5A

FIG. 5B

**FIG. 5C**

Cell Survival (%)
Target/GAPDH mRNA ratio (%)

MAP2K1 1
MAP2K1 2
MAP2K1 3
MAP2K1 4
MAP2K1 pool

MAP2K2 1
MAP2K2 2
MAP2K2 3
MAP2K2 4
MAP2K2 pool

LIPID
UNTRETED

LIPID (MAP2K1)
UNTREATED (MAP2K1)
LIPID (MAP2K2)
UNTREATED (MAP2K2)

0  20  40  60  80  100  120  140  160

FIG. 6A

FIG. 6B

FIG. 6C

**Controls**

T2

T1

HeLa

siRNA-
RISC-Free

→ siRNA-RF + pEGFP → Visualize (B,C)

↘ siRNA-EGFP+ pEGFP → Visualize (D,E)

HeLa

siRNA-
eIF2C2

→ siRNA-RF + pEGFP → Visualize (F,G)

↘ siRNA-EGFP+ pEGFP → Visualize (H,I)

**Experiment**

T1                     T2

HeLa    siRNA-
RISC-Free        Toxic siRNA    → Viability study (K)

HeLa    siRNA-
eIF2C2           Toxic siRNA    → Viability study (K)

**FIG. 7A**

EP 1 814 895 B1

# Control siRNA

# eIF2C2 siRNA

**Control siRNA**

**Control siRNA**

**FIG. 7B**

**FIG. 7C**

**FIG. 7F**

**FIG. 7G**

**FIG. 7D**

**EGFP siRNA**
**FIG. 7E**

**FIG. 7H**

**EGFP siRNA**
**FIG. 7I**

EP 1 814 895 B1

**FIG. 7J**

FIG. 8

**Cell Viability**

**Lipid concentration (ug/100uL)**

Legend: □ 0nM  □ 62.5nM  ▣ 125nM  □ 250nM

**Cell line A549**

**FIG. 9A**

# Gene Silencing

Cell line A549

FIG. 9B

**Cell Viability**

Alamar Blue (treated/untreated) — y-axis scale: 1.2, 1, 0.8, 0.6, 0.4, 0.2, 0

x-axis (Lipid concentration (ug/100uL)): 0.125, 0.25, 0.5 — Oligofectamine; 0.125, 0.25, 0.5 — DF1; 0.125, 0.25, 0.5 — T Bio

Legend: □ 0nM  □ 62.5nM  ▦ 125nM  □ 250nM

**Cell line 3T3L1**

**FIG. 10A**

EP 1 814 895 B1

**Gene Silencing**

Cell line 3T3L1

FIG. 10B

EP 1 814 895 B1

mRNA Knockdown by Cyclo3 siRNA with DF1 and Different Rehydration Media

FIG. 11A

**FIG. 11B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63032004 P
- US 67816505 P
- US 20060166234 A
- US 20060115461 A
- US 20060110829 A
- US 20060110766 A
- US 5811274 A, Palsson
- US 5804431 A, Palsson
- US 6544790 A, Sabatini
- US 20020006664 A, Sabatini
- US 2003070642 A, Caldwell
- US 71433303 A
- US 20070031844 A
- US 0336787 W
- WO 2004045543 A2
- US 94089204 A
- US 20050255487 A
- US 0414885 W
- US 20050246794 A
- US 6902883 B
- US 6875578 B
- US 6759206 B
- US 6716588 B
- US 6671624 B
- US 6620591 B
- US 6573039 B
- US 6416959 B
- US 5989835 A
- US 542646 P
- US 60543640 P
- US 60572270 P
- US 0410343 W
- WO 2004090105 A
- US 60678165 B
- US 5674108 A
- US 5834439 A
- US 6110916 A
- US 6399663 B
- US 6716582 B
- WO 0012454 A
- WO 9742819 A
- US 678165 P

### Non-patent literature cited in the description

- RNA interference microarrays: High-throughput loss-of-function genetics in mammalian cells. **Silva J M et al.** PROCEEDINGS OF THE NATIONAL ACADEMY OF SCIENCES OF USA. NATIONAL ACADEMY OF SCIENCE, 27 April 2004, vol. 101, 6548-6552
- SureSilencing Array Plates VALIDATED GENE-SPECIFIC siRNA FOR PATHWAY PROFILING BY REVERSE TRANSFECTION USER MANUAL. Superarray Bioscience Corporation, 03 May 2004